(19) Europäisches Patentamt
European Patent Office
Office européen des brevets

(11) **EP 1 275 338 B1**

(12) **EUROPEAN PATENT SPECIFICATION**

(45) Date of publication and mention
of the grant of the patent:
**17.12.2008 Bulletin 2008/51**

(51) Int Cl.:
**A61B 5/00** (2006.01)  **A61B 1/05** (2006.01)

(21) Application number: **02014010.9**

(22) Date of filing: **26.06.2002**

(54) **Apparatus for obtaining fluorescence images, and computer executable program therefor**

Gerät zur Herstellung von fluorezierenden Bildern und computerausführbares Programm dafür

Appareil d'obtention des images fluorescentes et programme exécutable par ordinateur correspondant

(84) Designated Contracting States:
**DE FR GB**

(30) Priority: **29.06.2001 JP 2001199131**
**27.03.2002 JP 2002089107**

(43) Date of publication of application:
**15.01.2003 Bulletin 2003/03**

(60) Divisional application:
**05002895.0 / 1 535 568**
**05002909.9 / 1 535 569**

(73) Proprietor: **FUJIFILM Corporation**
**Minato-ku**
**Tokyo (JP)**

(72) Inventors:
• **Sendai, Tomonari,**
**c/o Fuji Photo Film Co., Ltd.**
**Ashigarakami-gun,**
**Kanagawa-ken (JP)**
• **Hayashi, Katsumi,**
**c/o Fuji Photo Film Co., Ltd.**
**Ashigarakami-gun,**
**Kanagawa-ken (JP)**

(74) Representative: **Klunker . Schmitt-Nilson . Hirsch**
**Winzererstrasse 106**
**80797 München (DE)**

(56) References cited:
WO-A-99/37204          US-A- 4 773 097
US-A- 5 131 398          US-A- 5 294 799
US-A- 5 769 792

EP 1 275 338 B1

**Description**

BACKGROUND OF THE INVENTION

Field of the Invention

The present invention relates to according to the preamble an apparatus for obtaining fluorescence images by projecting an of claim 1 and a program for causing a computer to execute the fluorescence image obtaining method, according to the preamble of claim 4.

Description of the Related Art

Fluorescence detection apparatuses have been proposed that make use of the fact that the intensity of the fluorescence emitted from a normal tissue differs from the intensity of the fluorescence emitted from a diseased tissue when a target subject (i.e., a living tissue) is irradiated by an excitation light within an excitation wavelength range of the intrinsic fluorophores of the target subject. By detecting the fluorescence emitted from a target subject upon irradiation thereof by an excitation light within a predetermined wavelength range, the location and range of penetration of a diseased tissue is discerned.

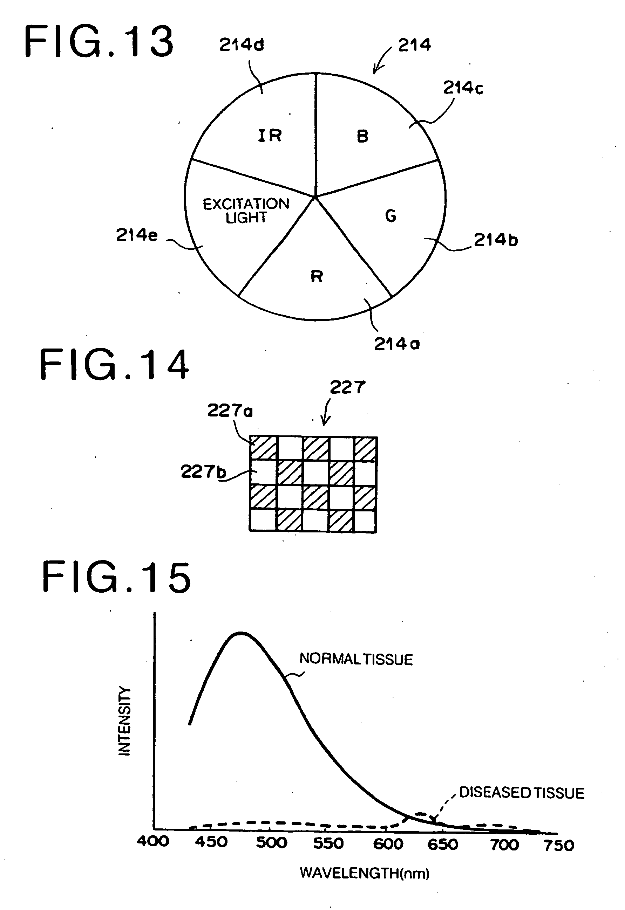

Normally, when a target subject is irradiated by an excitation light, because a high-intensity fluorescence is emitted from a normal tissue, as shown by the solid line in Fig. 15, and a weak-intensity fluorescence is emitted from a diseased tissue, as shown by the broken line in Fig. 15, by measuring the intensity of the fluorescence emitted from the target subject, it can be determined whether the target subject is in a normal or a diseased state.

Further, methods of imaging fluorescence by use of an imaging element or the like, and displaying a diagnostic fluorescence image corresponding to the intensity of the imaged fluorescence have been proposed. Here, because there is unevenness on the surface of a target subject, the intensity of the excitation light irradiating the target subject is not of a uniform intensity across the entirety of the surface thereof. Further, although the intensity of the fluorescence emitted from the target subject is substantially proportional to the intensity of the excitation light, the intensity of the aforementioned excitation light becomes weaker in inverse proportion to the square of the distance between the excitation light source and the target subject. Therefore, there are cases in which the fluorescence received from a diseased tissue located at a position closer to the excitation light source than a normal tissue is of a higher intensity than the fluorescence received from aforementioned normal tissue. Consequently, the state of the tissue of the target subject cannot be accurately discerned based solely on the data relating to the intensity of the fluorescence received from the target subject upon the irradiation thereof with an excitation light. In order to remedy the problems described above: a method of displaying an image based on the difference between the spectral forms representing the tissue states, that is, a method of dividing the intensities of two types of fluorescence intensities of two fluorescence images, each formed of fluorescence of a mutually different wavelength band (a narrow band near 480 nm and a wide band from near 430-730 nm) to obtain the ratio therebetween and displaying a computed image based on the obtained factor thereof as a fluorescence diagnostic image; a method of obtaining a value representing a fluorescence yield and displaying an image, that is, a method of projecting, as a reference light, onto a target subject a near-infrared light which is absorbed uniformly absorbed by tissues of a variety of tissue states, detecting the intensity of the reflected light reflected from the target subject upon the irradiation thereof by the reference light and dividing the intensity of the reflected light by the intensity of the fluorescence intensity to obtain the ratio therebetween, and displaying a computed image based on the obtained factor thereof as a fluorescence diagnostic image; and the like have been proposed. Further there have been proposed: a method of assigning color data to the factor of the intensities of the fluorescence of two different wavelength bands, or to the factor of a fluorescence intensity and the intensity of the reflected light reflected from the target subject upon the irradiation thereof by a reference light, to form a fluorescence diagnostic image wherein the diseased tissue of the target subj ect can be discerned from the difference in color within the fluorescence diagnostic image; a method of combining the color image representing the diseased tissue of the target subject by the difference in color and a brightness image formed by assigning brightness data to the intensity of the reflected light reflected from the target subject upon the irradiation thereof by the reference light to display a fluorescence diagnostic image also representing the contour of the surface of the target subject and imparting a three-dimensional sense thereof; such as those described in US Patent Nos. 5590660, 5647368, and Japanese Unexamined Patent Publication Nos. 9 (1997)-308604, 10(1998)-225436, and 2001-157658.

In this manner, by obtaining, displaying on a monitor and observing a fluorescence diagnostic image, an accurate determination can be made as to whether the target subject is in a normal state or a diseased state.

However, for cases in which a fluorescence diagnostic image is obtained of a target subject when blood, mucus, digestive fluids, saliva, foam, residue, and/or the like (hereinafter referred to as obstructing factors) is present on the target subject, because the obstructing factor is also obtained in the image at the same time, the fluorescence diagnostic image contains an image of the obstructing factor. Here, if an obstructing factor is present on the target subject, the intensity of the fluorescence emitted from the portion on which the obstructing factor is present becomes reduced, and fluorescence of a wavelength greater than or equal to 600 nm is emitted. Therefore, if a diagnosis is carried out using a fluorescence diagnostic image including an obstructing factor, there is a fear that the portion on which the obstructing factor is present will be judged to be a diseased tissue, even though said portion is a normal tissue. Hereinafter, the reasons whereby an obstructing factor leads to misdiagnosis will be explained.

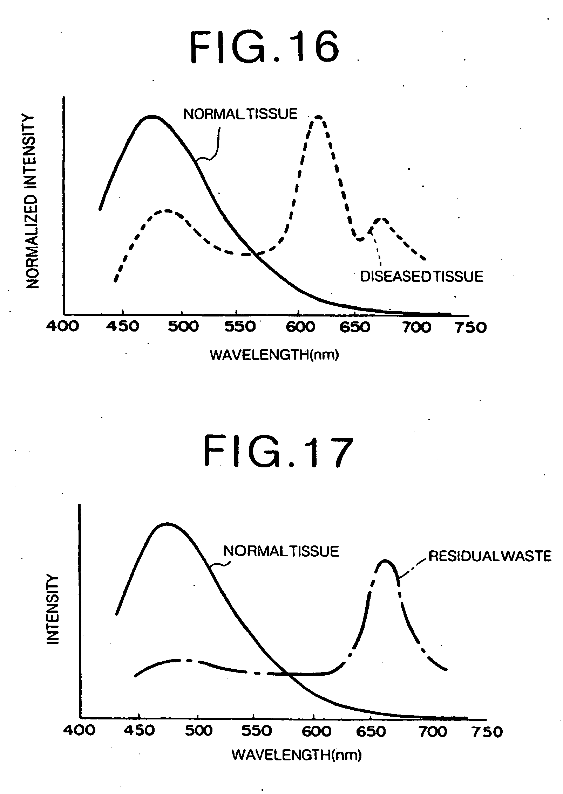

The spectrum of the fluorescence intensity emitted from the target subject upon the irradiation thereof by the excitation light is that shown in Fig. 15, and the normalized fluorescence intensity spectrum obtained by normalizing (causing the integral value over the entirety of the wavelength band to become 1) the aforementioned fluorescence intensity spectrum is that shown in Fig. 16. As shown in Fig. 15, the fluorescence intensity (an integral value over the entire wavelength band) emitted from a normal tissue and the fluorescence intensity emitted from a diseased tissue are clearly different. Further, as shown in Fig. 16, in the normalized fluorescence intensity spectrum, the relative intensity of the fluorescence of a wavelength near 480 nm emitted from the diseased tissue is reduced in comparison to that emitted from the normal tissue state, and the relative intensity of the fluorescence of a wavelength near 630 nm emitted from the diseased tissue is greater in comparison to that emitted from the normal tissue. Accordingly, it can be determined if the target subject is a normal tissue or a diseased tissue based on the fluorescence intensity and the normalized fluorescence intensity.

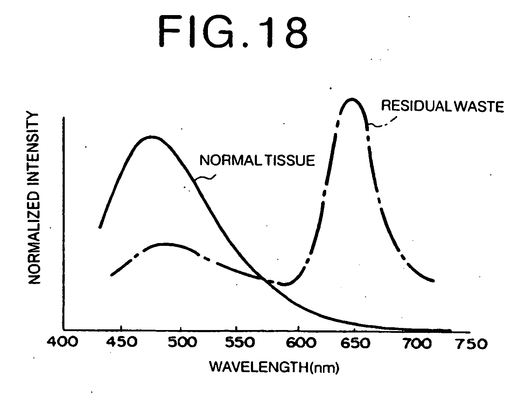

On the other hand, Fig. 17 shows the fluorescence intensity spectrum obtained of the fluorescence emitted from a residue obstructing factor upon the irradiation thereof by an excitation light, and Fig. 18 shows the normalized fluorescence intensity spectrum thereof. As shown in Fig. 17, in the case of residue, the fluorescence intensity spectrum thereof becomes approximately the same degree as that of the fluorescence emitted from a normal tissue; however, as shown in Fig. 18, with respect to the normalized fluorescence intensity spectrum of the fluorescence intensity spectrum of the fluorescence emitted from an obstructing factor, the relative intensity of the fluorescence of a wavelength near 480 nm is lower in comparison to that emitted from the normal tissue, and the relative intensity of the fluorescence of a wavelength near 670 nm is greater in comparison to that emitted from the normal tissue. Accordingly, according to the method wherein a computed image based on the factor of the intensities of two different types of fluorescence is employed as a fluorescence diagnostic image as described above, for example, in the case in which a fluorescence diagnostic image reflecting the form of the normalized fluorescence image intensity spectrum is obtained, because the pixel values of a portion in which a residue is present become the same pixel values as that of a diseased tissue, there is a fear that regardless of the fact that the tissue on which an obstructing factor is present is a normal tissue, said tissue will be diagnosed as being a diseased tissue.

In accordance with the pre-characterising part of claim 1 and claim 4, respectively, US-A-4,773,097 discloses a fluorescence image obtaining apparatus in which an obstructing regions detecting means is provided in order to detect optically disturbing components. To this end, an interference equation descriptive of an interference given by an optically disturbing component is derived. The disturbing component may be attributable to the optical interference from fluctuations in blood content. The interference equation is derived on the basis of paired data of a fluorescence image and a reflectance image.

US-A-5,769,729 discloses an endoscopic image system having a fluorescence diagnostic image obtaining means and means for obtaining a colour image which can be combined with the fluorescence images in order to improve the detection of various tissues.

SUMMARY OF THE INVENTION

The present invention has been developed in view of the forgoing circumstances, and it is an object of the present invention to provide a fluorescence image obtaining apparatus, and a program capable of causing a computer to execute a fluorescence image obtaining method; wherein, an accurate diagnosis can be performed using a fluorescence diagnostic image. This object is achieved by the features of claims 1 and 4, respectively.

The "fluorescence diagnostic image" can include: an image corresponding to the intensity of the fluorescence emitted from a target subject upon the irradiation thereof by an excitation light; an image representing the ratio between two types of fluorescence intensities obtained of two different wavelength bands; an image representing the ratio of the fluorescence intensity to the intensity of the reflected light reflected from the target subject upon the irradiation thereof by a reference light; an image formed by assigning color data to the ratio between the fluorescence intensities obtained of two different wavelength bands; an image formed by assigning color data to the ratio of the of fluorescence intensity to the reflectance intensity of the reflected light reflected from the target subject upon the irradiation thereof by a reference light; a synthesized image formed by combining a color image to which color data has been assigned and a brightness image obtained by assigning brightness data to the reflectance intensity of the reflected light reflected from the target subject upon the irradiation thereof by a reference light; or the like.

The referents of "color data" include, for example: the hue, saturation, and/or chromaticity (hue and saturation) of development color systems (HSB/HVC/Lab/Luv/La*b*/Lu*v* color spaces) or a mixed color system (an X,Y,Z color space); the color differences of a visible image signal representative of a TV signal (e.g., the IQ of the YIQ of an NTSC signal, the CbCr of an YCbCr, etc.); the combination ratio of a color signal (R, G, B or C, M, Y, G), etc.

The referents of "brightness data" include, for example: the luminosity or brightness of development color systems (HSB/HVC/Lab/Luv/La*b*/Lu*v* color spaces)or a mixed color system (an X, Y, Z color space); the brightness of a visible image signal representative of a TV signal (e.g., the Y of the YIQ of an NTSC signal, the CbCr of an YCbCr, etc.); etc.

The referents of "obstructing regions" are the regions representing locations of the target subject on which an obstructing factor such as blood, mucus, digestive fluids, saliva, foam, residue, and/or the like is present. The obstructing regions are regions of which there is a high probability of the misdiagnosis thereof as a diseased tissue, regardless of the fact that the tissue represented therein is in a normal tissue state. Note that according to the present invention, the regions of a fluorescence diagnostic image corresponding to obstructing regions as well as the regions of a standard image corresponding to obstructing regions are referred to as obstructing regions.

Preferred embodiment are defined by the dependent claims.

Note that according to the present invention, a white light can be projected onto the target subject and a standard image of said target subject can be obtained based on the reflected light obtained from said target subject upon the irradiation thereof by the white light, and

the obstructing regions included therein can be detected based on the color data of the standard image.

The fluorescence intensity and the computed fluorescence value representing the ratio between a plurality of fluorescence intensities obtained of different wavelength bands can be used as the fluorescence data.

The fluorescence intensity and the computed fluorescence value representing the ratio between a plurality of fluorescence intensities obtained of different wavelength bands can be used as the fluorescence data, and in this case, based on any one of the color data, the fluorescence intensity, or the fluorescence data (e.g., the color data) a first suspected obstructing region of the target subject can be detected, and

based on one of the data other than that employed in the detection of said first suspected obstructing region (e.g., the fluorescence intensity), a second suspected obstructing region of the target subject can be detected, and

based on one of the data other than that employed in the detection of said second suspected obstructing region (e.g., the computed fluorescence value), the obstructing regions can be detected.

The expression "exceptional display process" refers to a process enabling the display of the fluorescence diagnostic image in a manner wherein each image of an obstructing region included therein can be recognized as such at a glance. More specifically, the images of the obstructing regions can be processed so as to be of a color not appearing in any of the images of the other regions. For example, if the fluorescence diagnostic image is an image having chromatic color, the images of the obstructing regions can be regions having achromatic color, or conversely, if the fluorescence diagnostic image is an image having achromatic color, the images of the obstructing regions can be regions having chromatic color; further, for a case in which the color of the fluorescence diagnostic image changes from a green through yellow color to red in correspondence to the change of the tissue state from normal to diseased, the images of the obstructing regions can be caused to be blue. Still further, the images of the obstructing regions can be caused to be the same color as the background, or transparent. In addition, the images of the regions included in the fluorescence diagnostic image other than the obstructing regions can be caused to be transparent. Also, although there are cases in which the portions regarded to be in the diseased state are indicated by an arrow mark, in this type of case, a process whereby arrow marks are not assigned to obstructing regions is included in the exceptional display processes.

Further, according to the fluorescence image obtaining apparatus of the present invention, it is preferable that an exceptional display process means for subjecting the obstructing regions of the fluorescence diagnostic image to exceptional display processes, and

a display means for displaying the fluorescence diagnostic image that has been subjected to said exceptional display processes be further provided.

Still further, according to the fluorescence image obtaining apparatus according to the present invention, it is preferable that a portion or the entirety of the fluorescence diagnostic image obtaining means be provided in the form of an endoscope to be inserted into the body cavity of a patient.

Note that the fluorescence image obtaining method may be provided as a program capable of causing a computer to execute said fluorescence image obtaining method.

According to the present invention, when a fluorescence diagnostic image is obtained, because the obstructing regions representing the obstructing factor present on the target subject are detected, by causing the obstructing regions to be of a color different from that of the other regions or removing the obstructing regions, etc. and displaying the fluorescence diagnostic image, the fear that an obstructing region will be diagnosed as a tissue in a diseased state is eliminated. Accordingly, an accurate diagnosis can be performed using the fluorescence diagnostic image.

Further, for cases in which a standard image is obtained based on the reflected light obtained from the target subject upon the irradiation thereof by a white light, the obstructing regions included within the standard image become a different color than the other regions. Accordingly, the obstructing regions can be accurately detected based on the color data of the standard image.

.Further, by subjecting the obstructing regions occurring in a fluorescence diagnostic image to an exceptional display process when the fluorescence diagnostic image is to be displayed, when the displayed fluorescence diagnostic image is displayed, the obstructing regions can be recognized as such at a glance. Accordingly, the fear that an obstructing region will be misrecognized as a tissue in a diseased state is eliminated, and the diagnosis can be performed more accurately using the fluorescence diagnostic image.

Still further, if a fluorescence diagnostic image in which the images of the regions other than the obstructing regions have been caused to be transparent is superposed over a standard image and displayed, when the displayed standard image is observed, the obstructing regions can be recognized as such at a glance. Accordingly, the fear that a tissue in a diseased state that appears within obstructing region will be overlooked is eliminated, and the accuracy with which the diagnosis can be performed using the fluorescence diagnostic image is improved a level.

BRIEF DESCRIPTION OF THE DRAWINGS

Figure 1 is a schematic drawing of the main part of a fluorescence endoscope apparatus implementing the fluorescence image obtaining apparatus according to the first embodiment of the present invention,
Figure 2 is a schematic drawing of a CYG filter,
Figure 3 is a schematic drawing of a switching filter,
Figure 4 is a flowchart of the operation of the first embodiment from the detection of the obstructing regions to the performance of the exceptional display process,
Figure 5 is a schematic drawing of the main part of a fluorescence endoscope apparatus implementing the fluorescence image obtaining apparatus according to the second embodiment of the present invention,
Figure 6 is a flowchart of the operation of the second embodiment from the detection of the obstructing regions to the performance of the exceptional display process,
Figure 7 is a schematic drawing of the main part of a fluorescence endoscope apparatus implementing the fluorescence image obtaining apparatus according to a variation of the second embodiment of the present invention,
Figure 8 is a schematic drawing of the main part of a fluorescence endoscope apparatus implementing the fluorescence image obtaining apparatus according to the third embodiment of the present invention,
Figure 9 is a flowchart of the operation of the third embodiment from the detection of the obstructing regions to the performance of the exceptional display process,
Figure 10 is a flowchart of the operation of the fourth embodiment from the detection of the obstructing regions to the performance of the exceptional display process,
Figure 11 is a flowchart of the operation of the fifth embodiment from the detection of the obstructing regions to the performance of the exceptional display process,
Figure 12 is a schematic drawing of the sixth embodiment of the present invention;
Figure 13 is a schematic drawing of a rotating filter,
Figure 14 is a schematic drawing of a mosaic filter,
Figure 15 is a graph illustrating the respective intensity distributions of the fluorescence intensity spectrum of a tissue in a normal state and a tissue in a diseased state,
Figure 16 is a graph illustrating the respective intensity distributions of the normalized fluorescence intensity spectrum of a tissue in a normal state and a tissue in a diseased state,
Figure 17 is a graph illustrating the respective intensity distributions of the fluorescence intensity spectrum of a tissue in a normal state and a residue, and
Figure 18 is a graph illustrating the respective intensity distributions of the normalized fluorescence intensity spectrum of a tissue in a normal state and a residue.

DESCRIPTION OF THE PREFERRED EMBODIMENTS

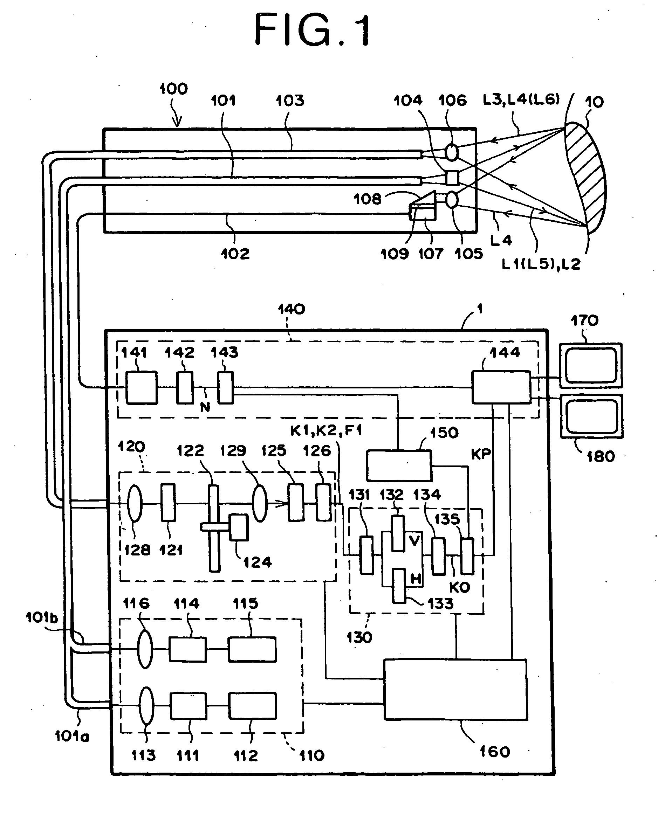

Hereinafter the preferred embodiments of the present invention will be explained with reference to the attached drawings. Fig. 1 is a schematic drawing of the main part of a fluorescence endoscope apparatus implementing the fluorescence image obtaining apparatus according to the first embodiment of the present invention. According to the fluorescence endoscope apparatus of the first embodiment of the present invention: the fluorescence emitted from a target subject upon the irradiation thereof by an excitation light is two-dimensionally detected by an image fiber; a narrow band fluorescence image formed of the fluorescence of a wavelength in the 430-530 nm wavelength band and a wide band fluorescence image formed of the fluorescence of a wavelength in the 530-730 nm wavelength band are obtained; a color image is formed based on the intensities of both fluorescence images, that is, on the factor of each corresponding pixel value of the narrow band fluorescence image and the wide band fluorescence image; an IR reflectance image is obtained of the reflected light reflected from the target subject upon the irradiation thereof by white light; a luminosity image is formed based on the light intensity of the IR reflectance image, that is, on the pixel value of each pixel of the IR reflectance image; the color image and the IR luminosity image are combined to form a synthesized image; and the synthesized image is displayed on a monitor as a fluorescence diagnostic image.

As shown in Fig. 1, the fluorescence endoscope apparatus according to the first embodiment of the present invention comprises: an endoscope insertion portion 100 for insertion into the primary nidus and suspected areas of disease of the patient; and an image signal processing portion 1.

The image signal processing portion 1 comprises: an illuminating unit 110 equipped with a light source for emitting a white light L1 (including a reference light L5) for obtaining a standard image and an IR reflectance image, and an excitation light L2 for obtaining a fluorescence image; an image obtaining unit 120 for obtaining two types of fluorescence images formed of different wavelength bands of fluorescence and an IR reflectance image of a target subject 10, and obtaining fluorescence image data K1, K2, and an IR reflectance image data F1; a fluorescence diagnostic image forming unit 130 for obtaining a factor between the corresponding pixel values of the respective fluorescence images represented by each of fluorescence image data K1 and K2 and obtaining a color image data H based on the obtained factor, forming a luminosity image data V based on the pixel value of the IR reflectance image represented by the IR reflectance image data F1, combining the color image data H and the luminosity image data V to form a fluorescence diagnostic image data, and further subjecting the fluorescence diagnostic image data to an exceptional display process, which is described below, to obtain a processed fluorescence diagnostic image data KP representing a processed fluorescence diagnostic image; an image processing unit 140 for subjecting the standard image represented by the standard image data N and the processed fluorescence diagnostic image represented by the processed fluorescence diagnostic image data KP to the processes required to display said images as visible images; an obstructing region detecting unit 150 for detecting the obstructing regions, which are described below; a controller 160 connected to each of the above units for controlling the operation timings thereof; a monitor 170 for displaying the normal image data N processed by the 'image process portion 140 as a visible image; and a monitor 180 for displaying the processed fluorescence diagnostic image data KP processed by the image process portion 140 as a visible image.

The endoscope insertion portion 100 is provided with a light guide 101 extending internally to the distal end thereof, A CCD cable 102, and an image fiber 103. An illuminating lens 104 and an objective lens 105 are provided at the distal end of the light guide 101, that is, at the distal end of the endoscope insertion portion 100. Further, the image fiber 103 is a quartz glass fiber, and is provided at the distal end thereof with a condensing lens 106. A CCD imaging element 107 (not shown) which is provided with an on-chip color filter is connected to the distal end of the CCD cable 102, and a prism 108 is attached to the CCD imaging element 107. Still further, an RGB filter 109 provided with R, G, and B band filter elements corresponding to each pixel of the CCD imaging element 107 and which are distributed in a mosaic pattern is disposed between the CCD imaging element 107 and the prism 108. A white light guide 101a, which is a composite glass fiber, and an excitation light guide 101b, which is a quartz glass fiber are bundled to form the light guide 101 as an integrated cable. The white light guide 101a and the excitation light guide 101b are connected to the illuminating unit 110. One end of the CCD cable 102 is connected to the image processing unit 140, and one end of the image fiber 103 is connected to the image obtaining unit 120.

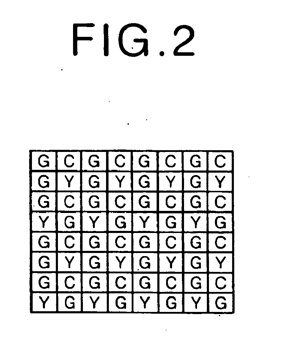

Note that a CYG filter, such as that shown in Fig. 2, which is formed of a C (cyan), a Y (yellow), and a G (green) band pass filters can be used instead of the RGB filter formed of the R, G, B band pass filters.

The illuminating unit 110 comprises: a white light source 111, which is a halogen lamp or the like, for emitting white light L1 (including a reference light L5 formed of near-infrared light) for obtaining standard images and IR reflectance images; a white light power source 112 which is electrically connected to the white light source 111; a white light condensing lens 113 for focusing the white light L1 emitted from the white light source 111; a GaN semiconductor laser 114 for emitting excitation light L2 for obtaining fluorescence images; an excitation light power source 115 which is electrically connected to the GaN semiconductor laser 114; and an excitation light condensing lens 116 for focusing the excitation light L2 emitted from the GaN semiconductor laser 114. Note that a reference light source that emits the reference light L5 can be provided separate from the white light source.

The image obtaining unit 120 comprises: a collimator lens 128 that guides the fluorescence L3 conveyed thereto via the image fiber 103; an excitation light cutoff filter 121 that cuts off light having a wavelength less than or equal to the 420 nm wavelength of the excitation light L2 from the fluorescence L3; a switching filter 122, in which three types of optical transmitting filters are combined; a filter rotating apparatus 124, which is a motor or the like, for rotating the switching filter 122; a condensing lens 129 for focusing the fluorescence L3 and the reflected light L6 transmitted by the switching filter 122; a CCD imaging element 125 for obtaining the fluorescence image and the IR reflectance image represented by the fluorescence L3 and the reflected light L6, respectively, focused by the condensing lens 129; and an A/D conversion circuit 126 for digitizing the image signals obtained by the CCD imaging element 125 to obtain two types of fluorescence image data K1, K2, and an IR reflectance image data F1.

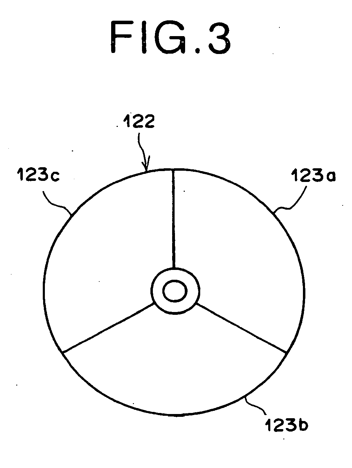

The configuration of the switching filter 122 is shown in Fig. 3. As shown in Fig. 3, the switching filter 122 comprises: an optical filter 123a, which is a band pass filter, that transmits light of a wavelength in the 430-730 wavelength band; an optical filter 123b, which is a band pass filter, that transmits light of a wavelength of 480 nm ±50nm; an optical filter 123c, which is a band pass filter, that transmits light of a wavelength in the 750-900 wavelength band. The optical filter 123a is an optical filter for obtaining a wide band fluorescence image; the optical filter 123b is an optical filter for obtaining a narrow band fluorescence image, and the optical filter 123C is an optical filter for obtaining an IR reflectance image. The switching filter 122 is controlled by the controller 160 via the filter rotating apparatus 124 so that the optical filter 123c is disposed along the optical path when the target subject 10 is being irradiated by the white light L1; and the optical filters 123a and 123b are alternately disposed along the optical path when the target subject 10 is being irradiated by the excitation light L2.

The fluorescence diagnostic image forming means 130 comprises: an image memory 131 for storing the two types of fluorescence image data K1, K2, and the IR reflectance image data F1 obtained by the A/D conversion circuit 126; a luminosity image computing portion 132, in which a look up table correlating the range of each pixel value of the IR reflectance image represented by the IR reflectance image data F1 to a luminosity in a Munsel display color system is stored, for referring to said look up table and obtaining a luminosity image data V from the IR reflectance image data F1; a hue computing portion 133, in which a look up table correlating the range of the factor between the two types of fluorescence images represented by the fluorescence image data K1, K2, to a hue in the hue circle of a Munsel display color system is stored, for referring to said look up table and forming a hue image data H from the factor between said fluorescence images; an image synthesizing portion 134 for combining the hue image data H and the luminosity image data V to form a fluorescence diagnostic image data KO representing a fluorescence diagnostic image; and an exceptional display processing portion 135 for subjecting the obstructing portions of the fluorescence diagnostic image to an exceptional display process to obtain a processed fluorescence diagnostic data KP.

The image memory 131 comprises a narrow band fluorescence image data storage region, a wide band fluorescence image data storage region, and an IR reflectance image data storage region, which are not shown in the drawing, wherein: the narrow band fluorescence image data K1 representing the narrow band fluorescence image obtained in the state wherein the excitation light L2 is being emitted and the narrow band fluorescence image optical filter 123a is disposed along the optical path of the fluorescence L3 conveyed by the image fiber 103 is recorded in the narrow band fluorescence image storage region; and the wide band fluorescence image data K2 representing the wide band fluorescence image obtained in the state wherein the excitation light L2 is being emitted and the wide band fluorescence image optical filter 123b is disposed along the optical path of the fluorescence L3 conveyed by the image fiber 103 is recorded in the wide band fluorescence image storage region. Further, the IR reflectance image data K1 representing the IR reflectance image obtained in the state wherein the reference light L5, that is the white light L1, is being emitted and the IR reflectance image optical filter 123c is disposed along the optical path of the reflected light L6, that is, the reflected light L4 conveyed by the image fiber 103, is recorded in the IR reflectance image storage region.

The exceptional display processing portion 135 performs an exceptional display process on the obstructing regions of the fluorescence diagnostic image represented by the fluorescence diagnostic image data K0. The exceptional display process is a process that causes the obstructing regions of the fluorescence diagnostic image to be displayed in a different form with respect to the other regions of the fluorescence diagnostic image. More specifically, the pixel values corresponding to the obstructing regions are converted to a color not appearing in any of the other regions of the fluorescence diagnostic image. For example, the pixels values of the obstructing regions can be converted to a blue color for a case in which the color change of the normal tissue and the diseased tissue of the target subject 10 range from green through yellow to red. Note that the color of the obstructing regions can be caused to be the same color as the background color, or the obstructing regions can be caused to be transparent. Alternatively, the images of the regions other than the obstructing regions included in the fluorescence diagnostic image can be caused to be transparent. Further, according to the current embodiment, because the fluorescence diagnostic image is a chromatic color image, the obstructing regions can also be caused to be non-chromatic in color. Note that for cases in which the fluorescence diagnostic image is a non-chromatic image, the obstructing regions can be cased to be chromatic.

Still further, the pixels within the obstructing regions can be displayed as gradation values. More specifically, the average color value Cave obtained of the target subject 10 and the standard deviation Cstd can be computed in advance, and the Mahalanobis distance Cm for the pixel value Cxy of each pixel of the obstructing regions can be obtained according to the following formula (1):

$$Cm = (Cxy - Cave)^2 / Cstd$$

The Mahalanobis distance Cm obtained by the formula (1) increases as the possibility of an obstructing region being of a color other than the average color of the target subject 10 becomes higher. Accordingly, by assigning a gradation value to the value of the Mahalanobis distance Cm, the obstructing region can be displayed as a gradation image corresponding to the magnitude of the possibility that said obstructing region represents an obstructing factor. Note that instead of the gradation display, it is possible to set and display the obstructing regions at the contour lines corresponding to the Mahalanobis distance Cm.

Further, for cases in which the portions regarded to be diseased tissue are indicated by an arrow mark, in the case of this type of display, a process whereby arrow marks are not assigned to obstructing regions is included in the exceptional display processes.

Note that the fluorescence diagnostic image forming unit 130 can be a unit for forming a processed fluorescence diagnostic image data KP based on the factor obtained between the corresponding pixel values of the fluorescence diagnostic images represented by the fluorescence diagnostic image data K1, K2, or based on the factor obtained by the performance of a division calculation between the pixel values of either of the fluorescence images and the pixel values of the IR reflectance image. Further, color data can be assigned to the factor obtained between the two fluorescence images or between one of the fluorescence images and the IR reflectance image, and the fluorescence diagnostic image data KP can be formed so as to represent the diseased state of the target subject 10 by the differences in color.

Further, for cases in which the IR reflectance image data F1 is used to form the fluorescence diagnostic image data K0, the R color data included in the standard image data N or the brightness data computed from the standard image data N can be used instead of the IR reflectance image data F1. Still further, for cases in which light of each of the colors R, G, and B is projected onto the target subject 10 and a standard image is obtained of the reflected light reflected from the target subject 10 thereupon, as described below, the color data based on the reflected red light can be used instead of the IR reflectance image data F1.

The image processing unit 140 comprises a signal processing circuit 141 for forming an analog standard image signal of the standard image, which is a color image, represented by the signal obtained by the CCD imaging element 107; an A/D converting circuit 142 for digitizing the standard image data formed in the signal processing circuit 141 to obtain a digital standard image data N; a standard image memory 143 for storing the standard image data N; and a video signal processing circuit 144 for converting the standard image data N outputted from the standard image memory 143 and the processed fluorescence diagnostic image data KP formed in the fluorescence diagnostic image forming unit 130 to video signals.

The obstructing regions detecting unit 150 is means that detects, based on the color data of the standard image represented by a standard image data N, obstructing regions representing regions in which an obstructing factor, such as blood, mucus, digestive fluids, saliva, foam, residue and/or the like is present on the target subject 10. Here, the color data can be that of, for example: the hue, saturation, and/or chromaticity (hue and saturation) of development color systems (HSB/HVC/Lab/Luv/La*b*/Lu*v* color spaces) or a mixed color system (an X,Y,Z color space); the color differences of a visible image signal representative of a TV signal (e.g., the IQ of the YIQ of an NTSC signal, the CbCr of an YCbCr, etc.); the combination ratio of a color signal (R, G, B or C, M, Y, G), etc.

More specifically, for the case in which the hue data is used as the color data, the standard image is of a specific hue range for cases in which the target subject 10 is a normal tissue and for cases in which the target subject 10 is a diseased tissue, respectively. On the other hand, for cases in which obstructing regions are present in the standard image, the hue of the obstructing factors is a hue other than that of either a normal tissue or a diseased tissue. Accordingly, the hue of each pixel of a standard image based on a standard image data N is computed, and a determination is made as to whether or not the hue of each pixel is the outside of a predetermined specific range; regions formed of pixels having a hue outside the predetermined specific range are detected as obstructing regions.

Further, for the case in which the chromaticity is used as the color data, the standard image is of a specific chromaticity range on the chromaticity chart for cases in which the target subject 10 is a normal tissue and for cases in which the target subject 10 is a diseased tissue, respectively. On the other hand, for cases in which obstructing regions are present in the standard image, the chromaticity of the obstructing factors is a chromaticity other than that of a normal tissue or a diseased tissue. Accordingly, the chromaticity of each pixel of a standard image based on a standard image data N is computed, and a determination is made as to whether or not the chromaticity of each pixel is the outside of a predetermined specific range; regions formed of pixels having a chromaticity outside the predetermined specific range are detected as obstructing regions.

Note that because the standard image data N is data formed of the data of each color R, G, B (or C, Y, G); the hue and chromaticity can be easily obtained if each color data is used. On the other hand, for the case in which the obstructing regions are detected based on the difference in color, the color difference signal can be computed from each color data R, G, B (or C, Y, G). However, according to the video signal processing circuit 144 according to the current embodiment, the standard image data N is converted to a video signal formed of brightness signals and color difference signals. Accordingly, if the color difference is to be used as the color data, the color difference obtained by the conversion of the standard image data N to a video signal by the video signal processing circuit 144 is used thereas; by the detection of the obstructing pixels by the obstructing region detecting unit 150, the step wherein the color difference is computed by the obstructing regions detecting unit 150 can be omitted.

Next, the operation of the first embodiment will be explained. First, the operation occurring when a standard image is to be obtained and displayed will be explained, followed by an explanation of the operations occurring when a reflectance image and a fluorescence image are to be obtained, and then an explanation of the operations occurring when the obstructing regions are detected, the fluorescence diagnostic image is synthesized, and the processed fluorescence diagnostic image is displayed will be explained.

According to the first embodiment of the present invention, the obtainment of a standard image, an IR reflectance image, and a fluorescence image are performed alternately in a temporal series. When the standard image and the IR reflectance image are to be obtained, the white light source power source 112 is activated, based on a signal from the controller 160, and white light L1 is emitted from the white light source 111. The white light L1 is transmitted by the white light condensing lens 113 and enters the white light guide 101a, and after being guided to the distal end of the endoscope insertion portion 100, is projected onto the target subject 10 from the illuminating lens 104.

The reflected light L4 of the white light L1 is focused by the objective lens 105, reflected by the prism 108, transmitted by the RGB filter 109, and focused on the CCD imaging element 107.

The signal processing circuit 141 forms an analog standard image signal, which represents a color image, from the reflected light L4 imaged by the CCD imaging element 107. The analog standard image signal is inputted to the A/D converting circuit 142, and after being digitized therein, is stored in the standard image memory 143. The standard image data N stored in the standard image memory 143 is converted to a video signal by the video signal converting circuit 144, and then input to the monitor 170 and displayed thereon as a visible image. The series of operations described above are controlled by the controller 160.

Meanwhile, at the same time, the reflected light L4 of the white light L1 (including the reflected light L6 of the reference light L5) is focused by the condensing lens 106, enters the distal end of the image fiber 103, passes through the image fiber 103 and is focused by the collimator lens 128, and is transmitted by the excitation light cutoff filter 121 and the optical filter 123c of the switching filter 122.

Because the optical filter 123c is a band pass filter that only transmits light of a wavelength in the 750-900 nm wavelength band, only the reflected light L6 of the reference light L5 is transmitted by the optical filter 123c.

The reflected light L6 transmitted by the optical filter 123c is received by the CCD imaging element 125. The analog IR reflectance image data obtained by the photoelectric conversion performed by the CCD imaging element 125 is digitized by the A/D converting circuit 126, and then stored as an IR reflectance image data F1 in the IR reflectance image region of the image memory 131 of the fluorescence image forming unit 130.

Next, the operation occurring when the fluorescence image is to be obtained will be explained. The excitation light source power source 115 is activated, based on a signal from the controller 160, and a 410 nm wavelength excitation light L2 is emitted from the GaN type semiconductor laser 114. The excitation light L2 is transmitted by the excitation light condensing lens 116 and enters the excitation light guide 101b, and after being guided to the distal end of the endoscope insertion portion 100, is projected onto the target subject 10 from the illuminating lens 104.

The fluorescence L3 emitted from the target subject 10 upon the irradiation thereof by the excitation light L2 is focused by the condensing lens 106, enters the distal end of the image fiber 103, passes through the image fiber 103 and is focused by the collimator lens 128, and is transmitted by the excitation light cutoff filter 121 and the optical filters 123a and 123b of the switching filter 122.

Because the optical filter 123a is a band pass filter that only transmits light of a wavelength in the 430-730 nm wavelength band, the fluorescence L3 transmitted by the optical filter 123a represents a wide band fluorescence image. Because the optical filter 123b is a band pass filter that only transmits light of a wavelength of $480 \pm 50$ nm, the fluorescence L3 transmitted by the optical filter 123b represents a narrow band fluorescence image.

The fluorescence L3 representing the narrow band fluorescence image and the wide band fluorescence image is received by the CCD imaging element 125, photoelectrically converted thereby, digitized by the A/D converting circuit 126, and then stored as a wide band fluorescence image data K1 in the wide band fluorescence image region and a narrow band fluorescence image data K2 the narrow band fluorescence image region of the image memory 131 of the fluorescence image forming unit 130.

Hereinafter the operation occurring when a processed fluorescence diagnostic image data KP is to be formed by the fluorescence diagnostic image forming unit 130 will be explained. First, the luminosity image computing portion 132 determines, utilizing the signal charge and a look up table, a luminosity occurring in a Munsel display color system for each pixel value of the IR reflectance image represented by the IR reflectance image data F1 to obtain a luminosity image data V, and outputs said luminosity image data V to the image synthesizing means 134.

The hue computing portion 133 of the fluorescence diagnostic image forming unit 130 divides the pixel value of each pixel of the narrow band fluorescence image represented by the narrow band fluorescence image data K2 by the pixel value of each corresponding pixel of the wide band fluorescence image represented by the in the wide band fluorescence image data K1 stored in the image memory 131 to obtain the respective factors thereof, and obtains, utilizing said factors and a prerecorded lookup table, a hue occurring in a Munsel display color system to obtain a hue image data H, and outputs the hue image data H to the image synthesizing portion 134.

The image synthesizing portion 134 synthesizes the hue image data H and the luminosity image data V to form a fluorescence diagnostic image K0 representing a fluorescence diagnostic image. Note that for cases in which the image is to be displayed in color, the image is displayed as a three color image; because the hue, luminosity, and saturation are required, when the image is synthesized, the largest values of the hue and the luminosity are obtained as the saturation S occurring in a Munsel display color system. Note that the fluorescence diagnostic image data K0 is subjected to an RGB conversion process, and becomes an image representing each of color R, G, and B.

Meanwhile, the obstructing regions detecting unit 150 detects, based on the color data of the standard image represented by the standard image data N, the regions of the target subject 10 on which an obstructing factor is present. Then, the exceptional display process unit 135 of the fluorescence diagnostic image forming unit 130 subjects the obstructing regions of the fluorescence diagnostic image represented by the fluorescence diagnostic image data K0 to an exceptional display process to obtain a processed fluorescence diagnostic image data KP.

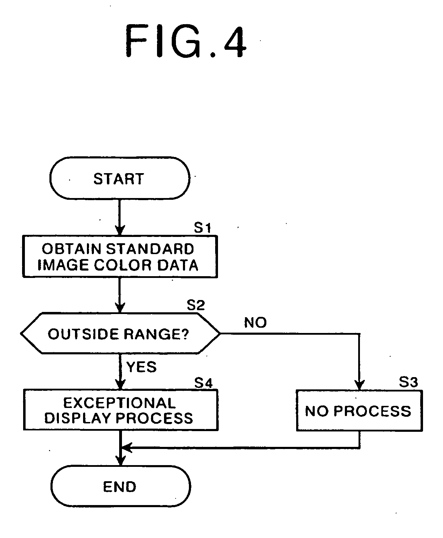

Hereinafter, the operations occurring from the detection of the obstructing regions to the performance of the exceptional display process will be explained utilizing the flowchart of Fig. 4. Fig. 4 is a flowchart of the operations occurring from the detection of the obstructing regions to the performance of the exceptional display process. First, the color data of each pixel of the standard image is computed by the obstructing regions detecting unit 150 (step S1), and then, a determination is made as to whether or not the color data obtained of each pixel of the standard image is outside a predetermined range (step S2). If the result of the determination made in step S2 is a negative, because the pixel of which a negative result is obtained is not a pixel representing an obstructing region, the corresponding pixel thereto of the fluorescence diagnostic image is subjected to no process whatsoever (step S3). If the result of the determination made in step S2 is a positive, the pixel of which a positive result is obtained is recognized as a pixel representing an obstructing region, and the corresponding pixel thereto of the fluorescence diagnostic image represented by the fluorescence diagnostic image data K0 is subjected to the exceptional display process by the exceptional display process portion 135 of the fluorescence diagnostic image forming unit 130 to obtain a processed fluorescence diagnostic image data KP (step S4).

The processed fluorescence diagnostic image data KP is outputted to the video signal processing circuit 144 of the image processing unit 140. The processed fluorescence diagnostic image data KP which has been converted to a video signal by the video signal processing circuit 144 is inputted to the monitor 180 and displayed thereon as a visible image. The obstructing regions of the processed fluorescence diagnostic image displayed on the monitor 180 have been subjected to the exceptional display process.

In this manner, the according to the current embodiment, because the obstructing regions within the fluorescence diagnostic image have been detected, by displaying on the monitor 180 the processed fluorescence diagnostic image obtained by subjecting the detected obstructing regions therein to an exceptional display process, the obstructing regions included in the fluorescence diagnostic image can be recognized in at a glance. Accordingly, an accurate diagnosis can be performed utilizing the fluorescence diagnostic image with no fear that obstructing regions will be diagnosed to be diseased tissue.

Further, if the exceptional display process consists of subjecting the images other than the obstructing regions occurring in the fluorescence diagnostic image to a process whereby said other regions are rendered transparent to obtain a processed fluorescence diagnostic image, and said obtained processed fluorescence diagnostic image is superposed over the standard image and displayed on the monitor 180, by observing said displayed standard image, the obstructing regions included therein can be recognized as such at a glance. Accordingly, the fear that a tissue in a diseased state appearing within an obstructing region will be overlooked is eliminated, and the accuracy with which the diagnosis can be performed using the fluorescence diagnostic image is improved a level.

Still further, if a configuration is adopted wherein the exceptional display process is capable of being selected, by use of an external switch or the like, from a plurality of exceptional display processes, the operational ease and versatility of the present apparatus can be improved a level. In when a standard diagnosis, for example, is to be performed, by displaying a fluorescence diagnostic image in which the obstructing regions included therein are of achromatic color, and the other portions thereof are of chromatic color, the misdiagnosis of obstructing regions as diseased tissue is prevented; on the other hand, by subjecting the images other than the obstructing regions occurring in the fluorescence diagnostic image to a process whereby said other regions are rendered transparent to obtain a processed fluorescence diagnostic image, and superposing said obtained processed fluorescence diagnostic image over the standard image immediately prior to concluding the diagnosis, the overlooking of diseased tissue included within the obstructing regions can be prevented.

Further, because the color of the obstructing regions differs from the color of the other regions, by detecting the obstructing regions based on the color data of the standard image, the obstructing regions can be detected accurately.

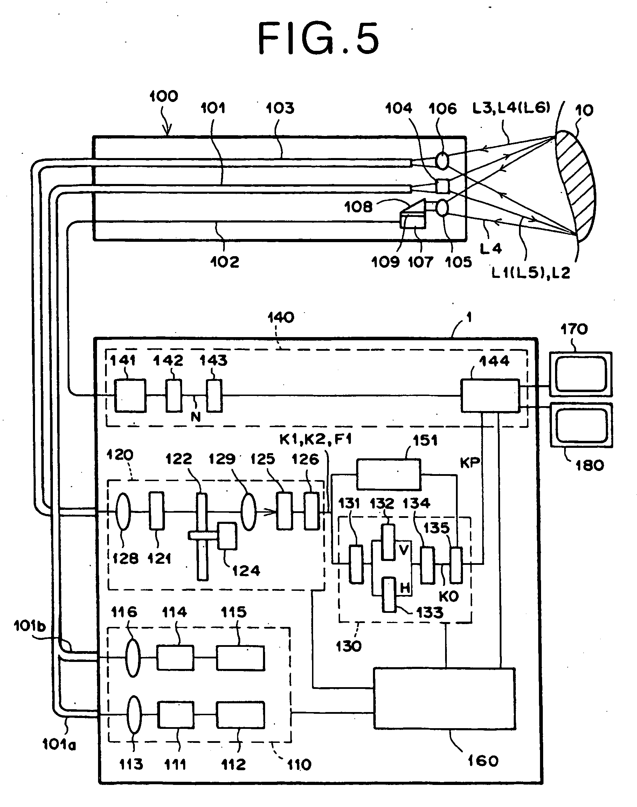

Next, the second embodiment of the present invention will be explained. Fig. 5 is a schematic drawing of the main part of a fluorescence endoscope apparatus implementing the fluorescence image obtaining apparatus according to the second embodiment of the present invention. Note that elements of the second embodiment that are the same as those of the first embodiment are likewise labeled, and further explanation thereof omitted. As shown in Fig. 5, the fluorescence endoscope apparatus according to the second embodiment of the present invention differs from that of the first embodiment in that instead of the obstructing regions detecting unit 150, which detects the obstructing regions based on the color data of the standard image, an obstructing regions detecting unit 151, which detects the obstructing regions based on the fluorescence intensity and the factor, that is the ratio between the pixel values of the corresponding pixels of two fluorescence images represented by two fluorescence image data K1, K2, respectively, is provided.

Here, the factor (hereinafter referred to as the computed fluorescence value) of the corresponding pixel values between the fluorescence images represented by the fluorescence image data K1 and K2 for an obstructing region is smaller than the value obtained of normal tissue and is close to the value obtained of a diseased tissue. On the other hand, the fluorescence intensity of an obstructing region is close to that of a normal tissue. Accordingly, the obstructing regions detecting unit 151 obtains the computed fluorescence value from the fluorescence image data K1 and K2, and makes a determination as to whether or not the obtained computed fluorescence value is less than or equal to a predetermined threshold value Th1. Next, a determination is made with respect to only the pixels of which the pixel value thereof has been determined to be less than or equal to the threshold value Th1, as to whether or not the fluorescence intensity thereof, that is, the fluorescence intensity of the pixel values of the fluorescence image represented by the fluorescence image data K1 or K2 is greater than or equal to a second threshold value Th2; the pixels of which the fluorescence intensity is determined to be greater than or equal to the threshold value Th2 are detected as obstructing regions. Note that instead of obtaining the computed fluorescence value itself, the obstructing regions detecting unit 151 can utilize the factor obtained of the corresponding pixels between the fluorescence images by the hue computing means 133 of the fluorescence diagnostic image forming means 130.

Next, the operation of the second embodiment will be explained. The operations occurring when the standard image is to be obtained, the standard image is to be displayed, the IR reflectance image is to be obtained, the fluorescence images are to be obtained, and the fluorescence diagnostic image is to be synthesized are the same as those occurring in the first embodiment; therefore, further explanation thereof is omitted. The operations occurring when the obstructing regions are to be detected and the processed fluorescence diagnostic image is to be displayed will be explained.

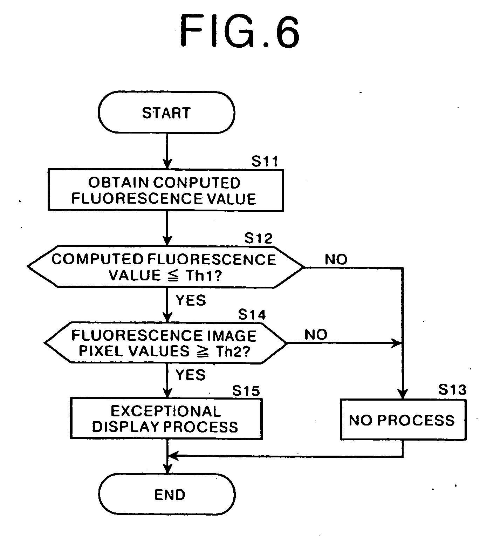

Fig. 6 is a flowchart of the operations from the detection of the obstructing regions to the performance of the exceptional display process according to the second embodiment. As shown in Fig. 6: first, the ratio between the fluorescence images represented by the fluorescence image data K1 and K2, that is, the computed fluorescence value therebetween, is obtained by the obstructing regions detecting unit 151 (step S11) ; then, a determination as to whether or not the computed fluorescence value of each pixel of the fluorescence images is less than or equal to the threshold value Th1 (step S12). If the result of the determination made in step S12 is a negative, because the pixel of which a negative result is obtained is not a pixel representing an obstructing region, the corresponding pixel thereto of the fluorescence diagnostic image is subjected to no process whatsoever (step S13) . If the result of the determination made in step S12 is a positive, because the possibility is high that the pixel of which a positive result is obtained is a pixel representing an obstructing region, a determination is made as to whether or not the fluorescence intensity thereof is greater than or equal to the threshold value Th2 (step S14). If the result of the determination made in step S14 is a negative, because the pixel of which a negative result is obtained is not a pixel representing an obstructing region, the corresponding pixel thereto of the fluorescence diagnostic image is subjected to no process whatsoever (step S13). If the result of the determination made in step S14 is a positive, the pixel of the fluorescence image represented by the respective fluorescence image data K1 or K2 is detected as an obstructing region, and the corresponding fluorescence diagnostic image data K0 is subjected to the exceptional display process by the exceptional display process portion 135 of the fluorescence diagnostic image forming unit 130 to obtain a processed fluorescence diagnostic image data KP (step S15).

The processed fluorescence diagnostic image data KP is outputted to the video signal processing circuit 144 of the image processing unit 140, and displayed on the monitor 180 as a visible image in the state in which the obstructing regions of the processed fluorescence diagnostic image have been subjected to the exceptional display process, in the same manner as occurred in the first embodiment.

Note that according to the second embodiment, although the determination performed in step 14 as to whether or not the pixel value is greater than or equal to the threshold value Th2 is performed only on the pixels of which the computed fluorescence value has been determined to be less than or equal to the threshold value Th1 is the step S12, the determination of step S14 can be performed first, and the computed fluorescence value obtained and the process of step S11 and the determination of S12 performed only for pixels that have returned a positive result in step S14. Further, the process of step S11, the determination of step S12, and the determination of step S14 can be performed in a series for all pixels, and the pixels of which the computed fluorescence value is less than or equal to the threshold value Th1 and which also have a pixel value greater than or equal to the threshold value Th2 detected as obstructing regions.

Further, according to the second embodiment, for cases in which the pixels within the obstructing regions are to be displayed with a display gradation: first, the average value FL ave of the fluorescence intensity obtained of the target subject 10 and the standard deviation FL std are computed in advance, and the Mahalanobis distance Fm of each pixel value FL xy included in the obstructing regions is obtained according to the formula (2) below.

$$Fm = (FLxy - Flave)^2 / FL \, std \quad (2)$$

The length of the Mahalanobis distance Fm obtained by use of the formula (2) becomes longer in proportion to an increase in the possibility that the fluorescence intensity is that of an obstructing region, which deviates from the average fluorescence intensity of the target subject 10. Accordingly, by assigning a gradation to the value of the Mahalanobis distance Fm, the obstructing regions can be displayed with a display gradation corresponding to the increase in the possibility that the obstructing region represents an obstructing factor. Note that instead of employing the display gradation, a contour line can be set in the obstructing regions in correspondence to the length of the Mahalanobis distance Fm, and displayed as a contour display.

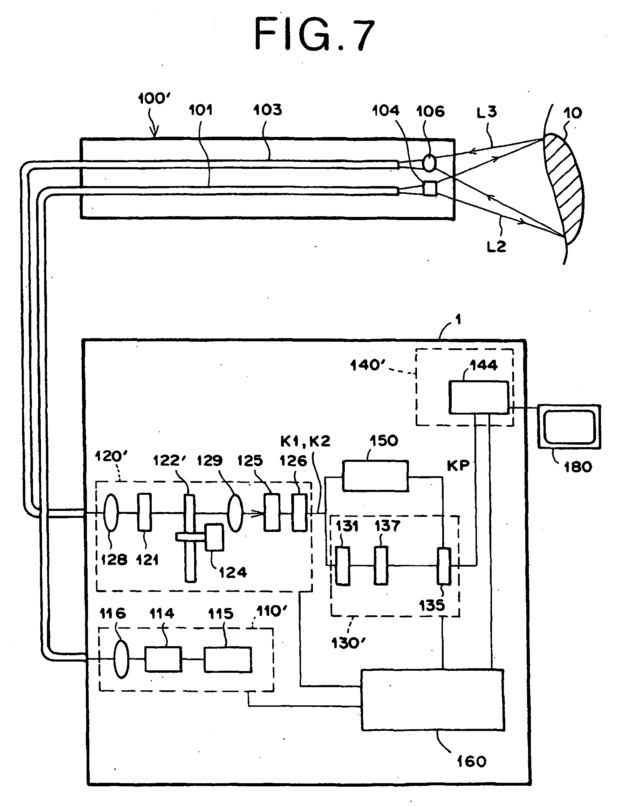

Further, according to the second embodiment described above, the obtainment of a standard image, an IR reflectance image, and fluorescence images is performed, however, as shown in Fig. 7, even if the fluorescence endoscope apparatus comprises only: an endoscope insertion portion 100' provided with only a light guide 101, an image fiber 103, an illuminating lens 104, and a condensing lens 106; an illuminating unit 110' provided with only a GaN type semiconductor laser 114, an excitation light power source 115, and an excitation light condensing lens 116; an image obtaining unit 120' provided with a switching filter 122', which has only an optical filters 123a and 123b, instead of the switching filter 122; a fluorescence diagnostic image forming means 130' formed only of an image memory 131, a computed fluorescence value obtaining portion 137, and an exceptional display process portion 135 for subjecting the obstructing portions of the computed image represented by the computed fluorescence values to obtain a processed fluorescence image data KP; an image process portion 140' formed provided with only a video processing circuit 144; a controller 160; and a monitor 180 for displaying the fluorescence diagnostic image; wherein, only fluorescence images are obtained and the computed fluorescence values thereof obtained, and said computed fluorescence values displayed as fluorescence diagnostic images, the obstructing regions can be subjected to the exceptional display process and displayed in the same manner as in the second embodiment.

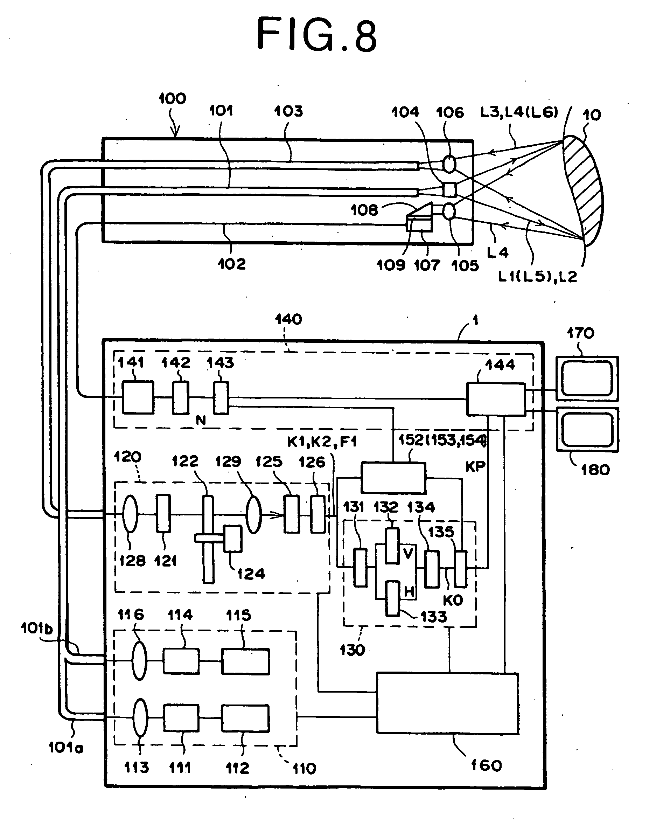

Next, the third embodiment of the present invention will be explained. Fig. 8 is a schematic drawing of the main part of a fluorescence endoscope apparatus implementing the fluorescence image obtaining apparatus according to the third embodiment of the present invention. Note that elements of the third embodiment that are the same as those of the first embodiment are likewise labeled, and further explanation thereof omitted. As shown in Fig. 8, the fluorescence endoscope apparatus according to the third embodiment of the present invention differs from that of the first embodiment in that instead of the obstructing regions detecting unit 150, which detects the obstructing regions based on the color data of the standard image, an obstructing regions detecting unit 152, which detects the obstructing regions based on the color data of the standard image and the fluorescence intensity, is provided.

Here, the color of an obstructing region is different from that of either that the normal or the diseased tissue. Further, the fluorescence intensity (i.e., the pixel values) of an obstructing region is close to that of normal tissue. Accordingly, the obstructing regions detecting unit 152 determines whether or not the color data of the standard image is outside a predetermined range, and then determines whether or not the pixel values of the fluorescence image corresponding to the pixels of which the color data is outside the predetermined range are greater than or equal to a predetermined threshold value Th3; the regions formed from the pixel values determined to be greater than or equal to the threshold value Th3 are detected as obstructing regions.

Next, the operation of the third embodiment will be explained. The operations occurring when the standard image is to be obtained, the standard image is to be displayed, the reflectance image is to be obtained, the fluorescence images are to be obtained, and the fluorescence diagnostic image is to be synthesized are the same as those occurring in the first embodiment; therefore, further explanation thereof is omitted. The operations occurring when the obstructing regions are to be detected and the processed fluorescence diagnostic image is to be displayed will be explained.

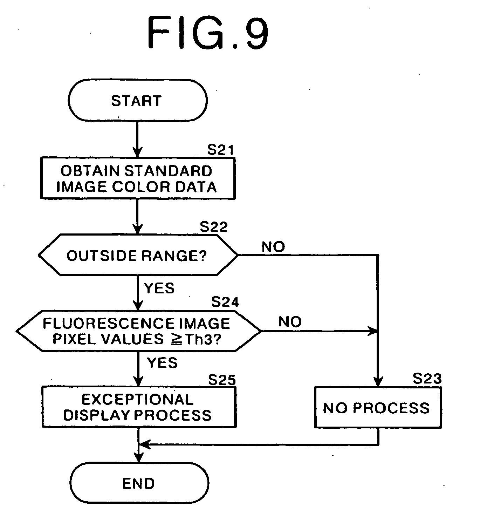

Fig. 9 is a flowchart of the operations from the detection of the obstructing regions to the performance of the exceptional display process according to the third embodiment. As shown in Fig. 9: first, the color data of each pixel of the standard image is computed (step S21) ; then, a determination as to whether or not the color data of each pixel of the standard image is outside the predetermined range (step S22). If the result of the determination made in step S22 is a negative, because the pixel of which a negative result is obtained is not a pixel representing an obstructing region, no process whatsoever is performed thereon (step S23). If the result of the determination made in step S22 is a positive, because the possibility is high that the pixel of which a positive result is obtained represents an obstructing region, a determination is made as to whether or not the pixel value of the corresponding pixel of the fluorescence image is greater than or equal to the threshold value Th3 (step S24). If the result of the determination made in step S24 is a negative, because the pixel of which a negative result is obtained is not a pixel representing an obstructing region, no process whatsoever is performed thereon (step S23). If the result of the determination made in step S24 is a positive, the pixel of which the positive result was returned is recognized as representing an obstructing region, and the corresponding fluorescence diagnostic image data K0 is subjected to the exceptional display process by the exceptional display process portion 135 to obtain a processed fluorescence diagnostic image data KP (step S35).

The processed fluorescence diagnostic image data KP is outputted to the video signal processing circuit 144 of the image processing unit 140, and displayed on the monitor 180 as a visible image in the state in which the obstructing regions of the processed fluorescence diagnostic image have been subjected to the exceptional display process, in the same manner as occurred in the first embodiment.

Note that according to the third embodiment, although the determination performed in step 24 as to whether or not the pixel value of the fluorescence image is greater than or equal to the threshold value Th3 is performed only on the pixels of which the color data has been determined to be outside the predetermined range in the step S22, the determination of step S24 can be performed first, and the color data obtained and the determination of S22 performed only for pixels that have returned a positive result in step S24. Further, the determination of step S22 and the determination of step S24 can be performed in a series for all pixels, and the pixels of the standard image of which the color data is outside the predetermined range and the corresponding pixels in the fluorescence image which also have a pixel value greater than or equal to the threshold value Th3 can be detected as obstructing regions.

Note that according to the third embodiment, for cases in which the pixels within the obstructing regions are to be displayed with a display gradation or as a contour line: first, using formula (1) or formula (2), the Mahalanobis distances Cm and Fm are obtained, and a display gradation can be assigned thereto or a contour line set therefor. Further, as shown in the formula (3) below, the Mahalanobis distances Cm and Fm can be subjected to a weighted addition process to obtain a total distance Gm, and a display gradation can be assigned to the total distance Gm, or a contour line set corresponding to the total distance Gm:

$$Gm = \alpha \cdot Cm + \beta \cdot Fm \quad (3)$$

where $\alpha$ and $\beta$ are weighing coefficients.

Next, the fourth embodiment of the present invention will be explained. The fluorescent endoscope according to the fourth embodiment differs from the fluorescence endoscope apparatus according to the third embodiment shown in Fig. 8, in that instead of the obstructing regions detecting unit 152, an obstructing regions detecting unit 153, which detects the obstructing regions based on the color data of the standard image and the ratio, that is the factor obtained between the corresponding pixels of the fluorescence images represented by two fluorescence image data K1, K2, is provided.

Here, the color of an obstructing region is different from that of either that the normal or the diseased tissue. Further, the factor (hereinafter referred to as the computed fluorescence value) obtained between the corresponding pixels of the fluorescence images represented by the fluorescence image data K1, K2 for an obstructing region is smaller than the value obtained of normal tissue and is close to that obtained of a diseased tissue. Accordingly, the obstructing regions detecting unit 153 determines whether or not the color data of the standard image is outside a predetermined range, then obtains the computed fluorescence value from, the fluorescence image data K1 and K2 only for the pixels of the fluorescence image corresponding to the pixels that have been determined to be outside the predetermined color range, and makes a determination as to whether or not the obtained computed fluorescence values are less than or equal to a predetermined threshold value Th4; the pixels of which the computed fluorescence value has been found to be less than or equal to the threshold value Th4 are detected as obstructing regions. Note that instead of obtaining the computed fluorescence value itself, the obstructing regions detecting unit 153 can utilize the factor obtained of the corresponding pixels between the fluorescence images by the hue computing means 133 of the fluorescence diagnostic image forming means 130.

Next, the operation of the fourth embodiment will be explained. The operations occurring when the standard image is to be obtained, the standard image is to be displayed, the IR reflectance image is to be obtained, the fluorescence images are to be obtained, and the fluorescence diagnostic image is to be synthesized are the same as those occurring in the first embodiment; therefore, further explanation thereof is omitted. The operations occurring when the obstructing regions are to be detected and the processed fluorescence diagnostic image is to be displayed will be explained.

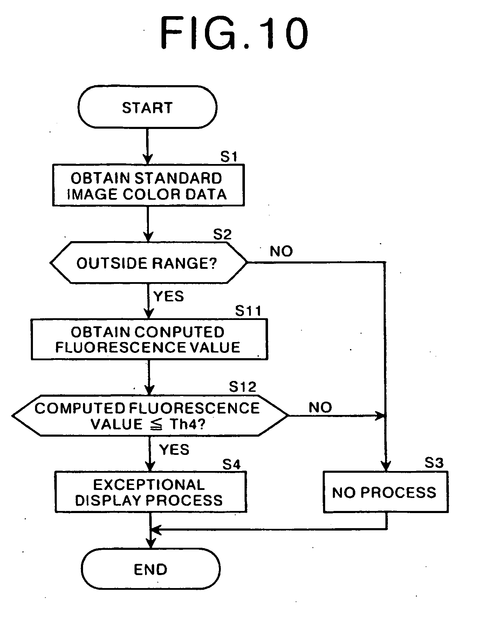

Fig. 10 is a flowchart of the operations from the detection of the obstructing regions to the performance of the exceptional display process according to the fourth embodiment. As shown in Fig. 10: first, the color data of each pixel of the standard image is computed (step S1); then, a determination is made as to whether or not the color data of each pixel of the standard image is outside the predetermined range (step S2). If the result of the determination made in step S2 is a negative, because the pixel of which a negative result is obtained is not a pixel representing an obstructing region, no process whatsoever is performed thereon (step S3). If the result of the determination made in step S2 is a positive, because the possibility is high that the pixel of which a positive result is obtained represents an obstructing region, the factor between the fluorescence images represented by the fluorescence image data K1 and K2, that is, the computed fluorescence value therebetween, is obtained only for the pixels corresponding to the pixels of the standard image which are outside the predetermined color range (step S11). Then, a determination as to whether or not the computed fluorescence value of each pixel of the fluorescence images is less than or equal to the threshold value Th4 (step S12). If the result of the determination made in step S12 is a negative, because the pixel of which a negative result is obtained is not a pixel representing an obstructing region, no process whatsoever is performed thereon (step S3). If the result of the determination made in step S12 is a positive, the pixel of which the positive result has been returned is recognized as representing an obstructing region, and the fluorescence diagnostic image data K0 corresponding thereto is subjected to the exceptional display process by the exceptional display process portion 135 of the fluorescence diagnostic image forming unit 130 to obtain a processed fluorescence diagnostic image data KP (step S4).

The processed fluorescence diagnostic image data KP is outputted to the video signal processing circuit 144 of the image processing unit 140, and displayed on the monitor 180 as a visible image in the state in which the obstructing regions of the processed fluorescence diagnostic image have been subjected to the exceptional display process, in the same manner as occurred in the first embodiment.

Note that according to the fourth embodiment, although obtainment of the computed fluorescence value in step S11 and the determination performed in step 12 as to whether or not the computed fluorescence value is less than or equal to the threshold value Th4 is performed only on the pixels of which the color data has been determined to be outside the predetermined range in the step S2, the determination of step S11 and the process of step S11 can be performed first, and the color data obtained and the determination of S2 performed only for pixels that have returned a positive result in step S12. Further, the determination of step S2, the determination of step S11, and the determination of step S12 can be performed in a series for all pixels, and the pixels of the standard image of which the color data is outside the predetermined range and the pixels corresponding thereto in the fluorescence image which also have a computed fluorescence value less than or equal to the threshold value Th4 can be detected as obstructing regions.

Next, the fifth embodiment of the present invention will be explained. The fluorescent endoscope according to the fifth embodiment differs from the fluorescence endoscope apparatus according to the third embodiment shown in Fig. 8, in that instead of the obstructing regions detecting unit 152, an obstructing regions detecting unit 154, which detects the obstructing regions based on the color data of the standard image, the fluorescence intensity and the ratio, that is the factor obtained between the corresponding pixels of the fluorescence images represented by two fluorescence image data K1, K2, is provided.

Here, the color of an obstructing region is different from that of either that the normal or the diseased tissue. Further, the fluorescence intensity obtained of an obstructing region is close to that obtained of a normal tissue. Still further, the factor (hereinafter referred to as the computed fluorescence value) obtained between the corresponding pixels of the fluorescence images represented by the fluorescence image data K1, K2 for an obstructing region is smaller than the value obtained of normal tissue and is close to the value obtained of a diseased tissue. Accordingly, the obstructing regions detecting unit 154 determines whether or not the color data of the standard image is outside a predetermined range, determines whether or not the pixel values, corresponding to those of which the color data is outside the predetermined range, of the fluorescence image are greater than the predetermined threshold value Th5, obtains the computed fluorescence value from the fluorescence image data K1 and K2 only for the pixels having a pixel value greater than or equal to the threshold value Th5, and determines whether or not the obtained computed fluorescence values are less than or equal to a predetermined threshold value Th6; the pixels of which the computed fluorescence value has been found to be less than or equal to the threshold value Th6 are detected as obstructing regions. Note that instead of obtaining the computed fluorescence value itself, the obstructing regions detecting unit 154 can utilize the factor obtained of the corresponding pixels between the fluorescence images by the hue computing means 133 of the fluorescence diagnostic image forming means 130.

Next, the operation of the fifth embodiment will be explained. The operations occurring when the standard image is to be obtained, the standard image is to be displayed, the IR reflectance image is to be obtained, the fluorescence images are to be obtained, and the fluorescence diagnostic image is to be synthesized are the same as those occurring in the first embodiment; therefore, further explanation thereof is omitted. The operations occurring when the obstructing regions are to be detected and the processed fluorescence diagnostic image is to be displayed will be explained.

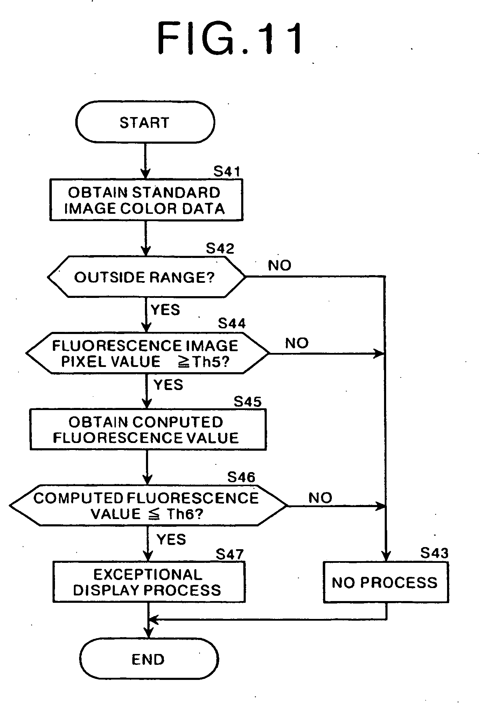

Fig. 11 is a flowchart of the operations from the detection of the obstructing regions to the performance of the exceptional display process according to the fifth embodiment. As shown in Fig. 11: first, the color data of each pixel of the standard image is computed (step S41); then, a determination as to whether or not the color data of each pixel of the standard image is outside the predetermined range (step S42). If the result of the determination made in step S42 is a negative, because the pixel of which a negative result is obtained is not a pixel representing an obstructing region, no process whatsoever is performed thereon (step S43). If the result of the determination made in step S42 is a positive, because the possibility is high that the pixel of which a positive result is obtained represents an obstructing region, a determination is made as to whether or not the pixels, corresponding to the pixels of the standard image which are outside the predetermined color range, of the fluorescence images are greater than or equal to the threshold value Th5 (step S44). If the result of the determination made in step S44 is a negative, because the pixel of which a negative result is obtained is not a pixel representing an obstructing region, no process whatsoever is performed thereon (step S43).

If the result of the determination made in step S44 is a positive, because the possibility is high that the pixel of which a positive result is obtained is a pixel representing an obstructing region, the factor, that is the computed fluorescence value between the fluorescence images represented by two fluorescence image data K1, K2 is obtained for only the pixels of which the pixel value is greater than or equal to the threshold Th5 (step S45) . Then, a determination is made as to whether or not the computed fluorescence values are less than or equal to the threshold value Th6 (step S46). If the result of the determination made in step S46 is a negative, because the pixel of which a negative result is obtained is not a pixel representing an obstructing region, the corresponding pixel thereto of the fluorescence diagnostic image is subjected to no process whatsoever (step S43). If the result of the determination made in step S46 is a positive, the pixel of which the positive result has been returned is recognized as an obstructing region, and the corresponding fluorescence diagnostic image data K0 is subjected to the exceptional display process by the exceptional display process portion 135 of the fluorescence diagnostic image forming unit 130 to obtain a processed fluorescence diagnostic image data KP (step S47).

The processed fluorescence diagnostic image data KP is outputted to the video signal processing circuit 144 of the image processing unit 140, and displayed on the monitor 180 as a visible image in the state in which the obstructing regions of the processed fluorescence diagnostic image have been subjected to the exceptional display process, in the same manner as occurred in the first embodiment.

Note that according to the fifth embodiment, the determination in step S44 as to whether or not the pixel values of the fluorescence images are greater than or equal to the threshold value Th5, the obtainment in step 45 of the computed fluorescence value for only the pixels of a pixel value greater than or equal to the threshold value Th5, and the determination as to whether or not the computed fluorescence values are less than or equal to the threshold value Th6 is performed only on the pixels of which the color data has been determined to be outside the predetermined range in the step S42; however, any of the steps can be performed first. For example, the determination of step S44, the determination of step S42, the process of S45, and the determination of step S46 can be performed in that order; or alternatively, the determination of step S44, the obtainment of step S45, the determination of step S46, and the determination of step S42 can be performed in that order. Further, the process of S45, the determination of step S46, the determination of step S42, and the determination of step S44 can be performed in that order; alternatively, the obtainment of step S45, the determination of step S46, the determination of step S44, and the determination of step S42 can be performed in that order.

Further, the determination of step S42, the determination of step S44, the obtainment of step 545, and the determination of step S46 can be performed as a series on all pixels; and the regions formed of the pixels of the standard image falling outside the predetermined color range, the pixels corresponding thereto of the fluorescence image which also have a pixel value greater than or equal to the threshold value Th5 and of which the computed fluorescence value thereof is less than or equal to the threshold value Th6 can be detected as obstructing regions.

Still further, after the determination of step S42 has been performed, the determination of step S44, followed by the process of step S45 and the determination of step S46 can be performed as a series; alternatively, after the determination of step S44 has been performed, the determination of step S42, followed by the process of step S45 and the determination of step S46 can be performed as a series. Further, after the process of step S45 and the determination of step S46 have been performed, followed by the determination of step S42 and the determination of step S44 can be performed as a series.

Note that according to the first through the fifth embodiments described above: when a determination is made as to whether or not the color data is outside the predetermined color range; a determination is made as to whether or not the pixel values of the fluorescence images are greater than or equal to a threshold value and/or as to whether or not the computed fluorescence value is less than or equal to a threshold value is to be made, the pixels of the standard image and/or the fluorescence images may be subjected to a thinning process. By thinning the pixels and performing the determinations in this manner, an increase in processing speed can be expected. Note that after these types of determinations have been performed, it is preferable that the determinations be performed without pixel thinning only for the detected obstructing regions.

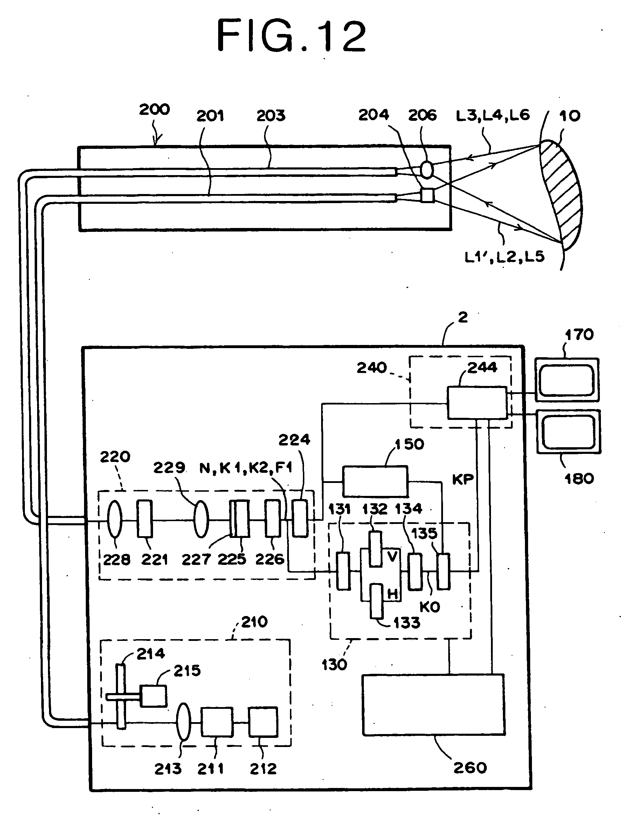

Further, according to the first through the fifth embodiments: it is also possible to project in sequence onto the target subject 10 R light, G light, B light, reference light, and excitation light to obtain a standard image, an IR reflectance image and fluorescence images. Hereinafter, this will be described as the sixth embodiment. Fig. 12 is a schematic drawing of the main part of a fluorescence endoscope apparatus implementing the fluorescence image obtaining apparatus according to the sixth embodiment of the present invention. Note that elements of the sixth embodiment that are the same as those of the first embodiment are likewise labeled, and further explanation thereof omitted. As shown in Fig. 12, the fluorescence endoscope apparatus according to the third embodiment of the present invention comprises an endoscope insertion portion 200 and an image signal processing portion 2.

The endoscope insertion portion 200 is provided with a light guide 201, an image fiber 203, an illuminating lens 204, an objective lens 205, and a condensing lens 206, which are the same as the light guide 101, an image fiber 103, an illuminating lens 104, an objective lens 105, and a condensing lens 106 configuring the endoscope insertion portion 100 of the first embodiment.

The image signal processing portion 2 comprises: an illuminating unit 210 for sequentially emitting R light, G light, B light (hereinafter collectively referred to as illuminating light L1'), a reference light L5, and an excitation light L2; an image obtaining unit 220 for imaging a standard image, two types of fluorescence images of two different wavelength bands, and an IR reflectance image, and obtaining a standard image data N, fluorescence image data K1 and K2, and an IR reflectance image data F1; a fluorescence diagnostic image forming means 130; an image processing unit 240 for subjecting the standard image represented by the standard image data N and the processed fluorescence diagnostic image represented by the processed fluorescence diagnostic image data KP to the processes required to display said images as visible images; an obstructing region detecting unit 150 for detecting the obstructing regions; a controller 260; a monitor 170; and a monitor 180.

The illuminating unit 210 comprises: a white light source 211, which is a halogen lamp or the like, for emitting white light; a white light power source 212 which is electrically connected to the white light source 211; a white light condensing lens 213; a rotating filter 214 for sequentially separating the colors or type of the emitted light into R light, G light, B light, reference light L5 and excitation light L2; and a motor 215 for rotating the rotating filter 214.

The configuration of the switching filter is shown in Fig. 13. As shown in Fig. 13, the switching filter 214 comprises filter elements 214a-214e that transmit: R light, G light, B light; near-infrared (IR) light of a wavelength in the 750-900 wavelength band; and excitation light L2 light of having a wavelength of 410 nm.

The image obtaining unit 220 comprises: a collimator lens 228 that guides the reflected light L4 of the R light, G, light, and B light, the reference light L5 the reflected light L6 and the fluorescence L3 conveyed thereto via the image fiber 203; an excitation light cutoff filter 221 that cuts off light having a wavelength less than or equal to the 420 nm wavelength of the excitation light L2 from the reflected light L4, L6, and the fluorescence L3; a condensing lens 229 for focusing the reflected light L4, L6 and the fluorescence L3; a CCD imaging element 225 for imaging the standard image, the IR reflectance image, and the fluorescence image represented by the reflected light L4, L6, and the fluorescence L3 respectively, which have been focused by the condensing lens 229; and an A/D conversion circuit 226 for digitizing the image signals obtained by the CCD imaging element 225 to obtain a standard image data N, an IR reflectance image data F1, and two types of fluorescence image data K1, K2; and a standard image memory 224 for recording a standard image data N.

Fig. 14 is a drawing of the configuration of the mosaic filter 227. As shown in Fig. 14, the mosaic filter 227 comprises wide band filter element 227a that transmits all light of a wavelength in the 400-900 nm wavelength band, and narrow band filter elements 227b that transmit light of a wavelength in the 430-530 nm wavelength band, which are combined alternately to form a mosaic pattern; each of the filter elements 227a and 227b are in a relation of a one-to-one correspondence with the pixels of the CCD imagining element 225.

Note that by the rotation of the rotating filter, the R light, the G light and B light, the IR near-infrared light and the excitation light are repeatedly projected onto the target subject 10. Here, while the R light, G light, B light, and reference light L5 are being projected onto the target subject 10, only the fluorescence image transmitted by the wide band filter elements 227a of the mosaic filter 227 is detected by the CCD imaging element 225, and while the excitation light L2is being projected onto the target subject, the respective fluorescence images passing through the wide band filter elements 227a and the narrow band filter elements 227b are detected by the CD imaging element 225.

The image processing unit 240 is provided with a video signal processing circuit 244, which is of the same configuration as the video signal processing circuit 144 of the first embodiment.

Next, the operation of the sixth embodiment will be explained. The operations occurring when the obstructing regions are to be detected and the processed fluorescence diagnostic image is to be displayed are the same as those occurring in the first embodiment; therefore, further explanation thereof is omitted. The operations occurring when the standard image is to be obtained, the standard image is to be displayed, and the IR reflectance image and the fluorescence images are to be obtained will be explained.

According to the endoscope apparatus of the sixth embodiment of the present invention, the obtainment of a standard image upon the irradiation of the target subject 10 with a R light, G light, and B light, the obtainment of an IR reflectance image, and the obtainment of a fluorescence image are performed alternately in a temporal series. Therefore, by causing the rotating filter 214 of the illuminating unit 210 is to rotate so that the white light emitted from the white light source 211 is transmitted by the rotating filter 214, the R light, the G light and B light, the IR near-infrared light and the excitation light are sequentially projected onto the target subject 10.

First, the operation occurring when a standard image is to be displayed will be explained. First, the R light is projected onto the target subject 10, and the reflected light L1 of the R light reflected from the target subject 10 is focused by the condensing lens 206, enters the distal end of the image fiber 203, passes through the image fiber 203 and is focused by the collimator lens 228, is transmitted by the excitation light cutoff filter 221, is focused by the condensing lens 229, transmitted by the wide band filter elements 227a of the mosaic filter 227, and is received by the CCD imaging element 225.

After the reflected light L4 of the R light received at the CCD imaging element 225 has been photoelectrically converted therein, and then converted to a digital signal by the A/D converting circuit 226 to obtain an R light image data, the R light image data is stored in the R light image data region recording region of the standard image memory 224.

After the passage of a predetermined period of time, the rotating filter 214 is caused to rotate to switch the filter element disposed along the optical path of the white light emitted from the white light source from the R light filter element 214a to the G light filter element 214b, and the G light image data is obtained according to the same operation described above. Further, after the passage of a predetermined period of time, the rotating filter 214 is caused to rotate so as to switch to the B light filter element 214c, and the B light image data is obtained. The G light image data and the B light image data are stored in the G light image data recording region and the B light image data recording region, respectively, of the standard image memory 224.

When the image data for the three colors have been stored in the standard image memory 224, said three images are synchronized and outputted simultaneously as a standard image data N to the video signal processing circuit 244. The video signal processing circuit 244 converts said inputted signals to video signals and outputs said video signals to the monitor 170, and said video signals are displayed thereon as a visible image.

Next, the operation occurring when a fluorescence image is to be obtained will be explained. The rotating filter 214 is again caused to rotate, based on a control signal from the controller 260, from the filter element 214d to the filter element 214e; wherein, the filter element 214e is positioned along the optical path of the white light emitted from the illuminating unit 210. In this manner, the excitation light L2 is projected onto the target subject 10.

The fluorescence L3 emitted from the target subject 10 upon the irradiation thereof by the excitation light L2 is focused by the condensing lens 206, is focused by the condensing lens 206, enters the distal end of the image fiber 203, passes through the image fiber 203 and is focused by the collimator lens 228, is transmitted by the excitation light cutoff filter 221, is focused by the condensing lens 229, transmitted by the wide band filter elements 227a and the narrow band filter element 227b of the mosaic filter 227, and is received by the CCD imaging element 225.

After the fluorescence L3 received at the CCD imaging element 225 has been photoelectrically converted for pixel each corresponding to the wide band filter elements 227a and the narrow band filter element 227b, and then converted to a digital signal by the A/D converting circuit 226 to obtain a wide band fluorescence image data K1 and a narrow band fluorescence image data K2, the wide band fluorescence image data K1 and the narrow band fluorescence image data K2 are stored in the wide band fluorescence image data recording region and the narrow band fluorescence image data recording region, respectively, of the image memory 131 of the fluorescence diagnostic image forming unit 130.

Then, in the same manner as occurs in the first embodiment, the image synthesizing portion 134 of the fluorescence diagnostic image forming means synthesizes a fluorescence diagnostic image data K0. Meanwhile, the obstructing regions detecting unit 150 detects, based on the color data of the standard image, the obstructing regions. The exceptional display process portion 135 subjects the detected obstructing regions are to an exceptional display process to obtain a processed fluorescence diagnosis image data KP. The processed fluorescence diagnosis image data KP is converted to video signals by the video signal processing circuit 244, inputted to the monitor 180, and displayed thereon as a visible image.

Note that the second through the fifth embodiments can also utilize, in the same manner as described above, an illuminating unit 220 and an image processing portion 240 instead of the illuminating unit 110, the image obtaining unit 120, and the image processing portion 240.

Further, according to the first through sixth embodiments described above, the CCD imaging element for obtaining fluorescence images has been provided within the image processing portion; however, a CCD imaging element equipped with the on-chip mosaic filter 227 shown in Fig. 14 can be disposed in the distal end of the endoscope insertion portion. In addition, if the CCD imaging element is a charge multiplying type CCD imaging element, such as that described in Japanese Unexamined Patent Publication No. 7 (1995) -176721, for amplifying the obtained signal charge, the obtainment of the fluorescence images can be performed at a higher sensitivity, and the noise component of the fluorescence images can be further reduced.

**Claims**

1. A fluorescence image obtaining apparatus comprising:

a fluorescence diagnostic image obtaining means (120) for obtaining, based on the fluorescence obtained from a target subject (10) upon the irradiation thereof by an illuminating light containing excitation light (L2), a fluorescence diagnostic image of a target subject (10), further comprising
an obstructing regions detecting means (150) for detecting the obstructing factors present on the target subject, such obstructing factors, e.g. blood, mucus, digestive fluids, saliva, foam, residue, and/or the like, reducing the intensity of the fluorescence emitted from the portion of the target on which an obstructing factor is present,

**characterized by** further comprising

a standard image obtaining means (140) for obtaining, based on the reflected light obtained from the target subject (10) upon the irradiation thereof by a white light (21), a standard image of the target subject (10), wherein said obstructing regions detecting means (150) is a means adapted for detecting the obstructing regions based on the color data of the standard image.

2. A fluorescence image obtaining apparatus as defined in claim 1, further comprising
an exceptional display process means (135) for subjecting the obstructing regions of the fluorescence diagnostic image to exceptional display processes, and
a display means (180) for displaying the fluorescence diagnostic image that has been subjected to said exceptional display processes.

3. A fluorescence image obtaining apparatus as defined in claim 1 or 2, wherein
a portion of the entirety of the fluorescence diagnostic image obtaining means (120) is provided in the form of an endoscope (100) to be inserted into the body cavity of a patent.

4. A program for causing a computer to execute a fluorescence image obtaining method, the program comprises the steps of:

projecting an illuminating light containing excitation light (L2) onto a target subject (10):

obtaining a fluorescence diagnostic image based on the fluorescence obtained from said target subject (10) upon the irradiation thereof by said light: and
detecting obstructing regions representing an obstructing factor present on the target subject, such obstructing factors, e.g. blood, mucus, digestive fluids, saliva, foam, residue, and/or the like, reducing the intensity of the fluorescence emitted from the portion of the target on which an obstructing factor is present,

**characterized by** the step of

projecting a white light (21) is onto the target subject to further obtain a standard image of said target subject is based on the reflected light obtained from said target subject (10) upon the irradiation thereof by the white light, wherein
said step of detecting obstructing regions is adapted to detect the obstructing regions included therein based on the color data of the standard image.

5. A program as defined in claim 4, wherein
the fluorescence intensity and/or the computed fluorescence value representing the ratio between a plurality of fluorescence intensities obtained of different wavelength bands are used as the fluorescence data.

6. A program as defined in claim 4 or 5, further comprising the steps of
subjecting the obstructing regions of the fluorescence diagnostic image to an exceptional display process, and
displaying the fluorescence diagnostic image subjected to said exceptional display process.

**Patentansprüche**

1. Fluoreszenzbild-Erstellungsvorrichtung, umfassend:

eine Fluoreszenz-Diagnosebild-Erstellungseinrichtung (120), um basierend auf der von einem Zielobjekt (10) bei dessen Bestrahlung mit einem Anregungslicht (L2) enthaltenden Beleuchtungslicht erhaltener Fluoreszenz ein Fluoreszenz-Diagnosebild eines Zielobjekts (10) zu erstellen, weiterhin umfassend:

eine Sperrzonen-Detektoreinrichtung (150) zum Detektieren der an dem Zielobjekt befindlichen Sperrfaktoren wie beispielsweise Blut, Schleim, Verdauungsflüssigkeiten, Speichel, Schaum, Rückstände und/oder dergleichen, welche die Intensität der Fluoreszenz vermindern, die von dem Teil des Ziels emittiert wird, auf dem sich ein sperrendes Mittel befindet,

**dadurch gekennzeichnet, dass** sie weiterhin umfasst:

eine Standardbild-Erstellungseinrichtung (140), die basierend auf dem von dem Zielobjekt (10) bei dessen Bestrahlung mit weißem Licht (21) erhaltenen reflektierten Licht ein Standardbild des Zielobjekts (10) erstellt, wobei

die Sperrzonen-Detektoreinrichtung (150) eine Einrichtung ist, die ausgebildet ist zum Nachweisen der Sperrzonen basierend auf den Farbdaten des Standardbilds.

**2.** Vorrichtung nach Anspruch 1, weiterhin umfassend

eine Ausnahmeanzeige-Verarbeitungseinrichtung (135), um die Sperrzonen des Fluoreszenz-Diagnosebilds Ausnahmeanzeige-Prozessen zu unterziehen, und

eine Anzeigeeinrichtung (180) zum Anzeigen des Fluoreszenz-Diagnosebilds, das den Ausnahmeanzeige-Prozessen unterzogen wurde.

**3.** Vorrichtung nach Anspruch 1 oder 2, bei der ein Teil der Gesamtheit der Fluoreszenz-Diagnosebild-Erstellungseinrichtung (120) in Form eines Endoskops (100) zum Einführen in den Körperhohlraum eines Patienten vorgesehen ist.

**4.** Programm zum Veranlassen eines Computers, ein Fluoreszenzbild-Erstellungsverfahren auszuführen, umfassend folgende Schritte:

Projizieren eines Anregungslicht (L2) enthaltenden Beleuchtungslichts auf ein Zielobjekt (10);

Erstellen eines Fluoreszenz-Diagnosebilds auf der Grundlage der Fluoreszenz, die von dem Zielobjekt (10) bei dessen Bestrahlung mit dem Licht erhalten wurde; und

Detektieren von Sperrzonen, die einen an dem Zielobjekt vorhandenen Sperrfaktor repräsentieren, so zum Beispiel Blut, Schleim, Verdauungsflüssigkeiten, Speichel, Schaum, Rückstände, und/oder dergleichen, die die Intensität der Fluoreszenz mindern, die von dem Teil des Ziels emittiert wird, auf dem sich ein Sperrfaktor befindet,

**gekennzeichnet durch** den Schritt:

Projizieren von weißem Licht (21) auf das Zielobjekt, um zusätzlich ein Standardbild des Zielobjekts basierend auf dem reflektierten Licht zu erstellen, welches von dem Zielobjekt (10) bei dessen Bestrahlung mit dem weißen Licht erhalten wird, wobei der Schritt des Detektierens von Sperrzonen dazu ausgebildet ist, die Sperrzonen basierend auf den Farbdaten des Standardbilds zu erfassen.

**5.** Programm nach Anspruch 4, bei dem die Fluoreszenzintensität und/oder der berechnete Fluoreszenzwert, der das Verhältnis zwischen mehreren aus unterschiedlichen Wellenlängenbändern erhaltenen Fluoreszenzintensitäten repräsentiert, als Fluoreszenzdaten verwendet werden.

**6.** Programm nach Anspruch 4 oder 5, weiterhin umfassend die Schritte:

die Sperrzonen des Fluoreszenz-Diagnosebilds werden einem Ausnahmeanzeige-Prozess unterzogen, und

das dem Ausnahmeanzeige-Prozess unterzogene Fluoreszenz-Diagnosebild wird zur Anzeige gebracht.

**Revendications**

**1.** Appareil d'obtention d'images fluorescentes comprenant :

un moyen d'obtention d'images de diagnostique fluorescentes (120) fournissant, sur la base de la fluorescence fournie par un sujet cible (10) lors de son irradiation par une lumière d'illumination contenant de la lumière d'excitation (L2), une image de diagnostique fluorescente d'un sujet cible (10), comprenant en outre

un moyen de détection de régions d'obstruction (150) détectant les facteurs d'obstruction présents sur le sujet cible, lesquels facteurs d'obstruction, par exemple du sang, du mucus, des fluides digestifs, de la salive, de la mousse, des résidus et/ou similaires, réduisant l'intensité de la fluorescence émise par la partie de la cible sur laquelle un facteur d'obstruction est présent,

**caractérisé en ce qu'**il comprend en outre :

un moyen d'obtention d'image standard (140) fournissant, sur la base de la lumière réfléchie fournie par le sujet cible (10) lors de son irradiation par une lumière blanche (21), une image standard du sujet cible (10), ledit moyen de détection de régions d'obstruction (150) étant un moyen adapté pour détecter les régions d'obstruction sur la base de données colorées de l'image standard.

2. Appareil d'obtention d'images fluorescentes selon la revendication 1, comprenant en outre :

un moyen de traitement exceptionnel d'affichage (135) permettant de soumettre les régions d'obstruction de l'image de diagnostique fluorescente à des traitements exceptionnels d'affichage, et
un moyen d'affichage (180) affichant l'image de diagnostique fluorescente qui a été soumise auxdits traitements exceptionnels d'affichage.

3. Appareil d'obtention d'images fluorescentes selon la revendication 1 ou 2, dans lequel
le moyen d'obtention d'images de diagnostique fluorescentes (120)est prévu, en partie ou en totalité, sous la forme d'un endoscope (100) destiné à être inséré dans une cavité corporelle d'un patient.

4. Programme permettant à un ordinateur d'exécuter un procédé d'obtention d'images fluorescentes, le programme comprenant les étapes consistant à :

projeter une lumière d'éclairage contenant de la lumière d'excitation (L2) sur un sujet cible (10) ;
obtenir une image de diagnostique fluorescente sur la base de la fluorescence fournie par ledit sujet cible (10) lors de l'irradiation de ce dernier par ladite lumière ; et
détecter les régions d'obstruction représentant un facteur d'obstruction présent sur le sujet cible, lesquels facteurs d'obstruction, par exemple du sang, du mucus, des fluides digestifs, de la salive, de la mousse, des résidus et/ou similaires, réduisant l'intensité de la fluorescence émise par la partie de la cible sur laquelle un facteur d'obstruction est présent,

**caractérisé par** l'étape consistant à :

projeter une lumière blanche (21) sur le sujet cible afin encore d'obtenir une image standard dudit sujet cible sur la base de la lumière réfléchie fournie par ledit sujet cible (10) lors de l'irradiation de ce dernier par la lumière blanche,
ladite étape de détection des régions d'obstruction étant adaptée à la détection des régions d'obstruction qui y sont présentes sur la base des données colorées de l'image standard.

5. Programme selon la revendication 4, dans lequel
l'intensité de fluorescence et/ou la valeur de fluorescence calculée représentant le rapport entre une pluralité d'intensités de fluorescence obtenues de différentes bandes de longueurs d'ondes sont utilisées comme données de fluorescence.

6. Programme selon la revendication 4, comprenant en outre les étapes consistant à :

soumettre les régions d'obstruction de l'image de diagnostique fluorescente à un traitement exceptionnel d'affichage, et
afficher l'image de diagnostique fluorescente soumise audit traitement exceptionnel d'affichage.

# FIG. 1

# FIG.2

| G | C | G | C | G | C | G | C |
|---|---|---|---|---|---|---|---|
| G | Y | G | Y | G | Y | G | Y |
| G | C | G | C | G | C | G | C |
| Y | G | Y | G | Y | G | Y | G |
| G | C | G | C | G | C | G | C |
| G | Y | G | Y | G | Y | G | Y |
| G | C | G | C | G | C | G | C |
| Y | G | Y | G | Y | G | Y | G |

# FIG.3

# FIG.4

```
                ╭─────────────────╮
                │      START       │
                ╰─────────────────╯
                         │
                         ▼           S1
                ┌─────────────────┐
                │ OBTAIN STANDARD  │
                │ IMAGE COLOR DATA │
                └─────────────────┘
                         │
                         ▼           S2
                ╱───────────────────╲        NO
                ╲  OUTSIDE RANGE?    ╱──────────────┐
                 ╲─────────────────╱               │
                         │ YES                      │
                         ▼           S4             ▼           S3
                ┌─────────────────┐      ┌─────────────────┐
                │   EXCEPTIONAL    │      │   NO PROCESS     │
                │ DISPLAY PROCESS  │      │                  │
                └─────────────────┘      └─────────────────┘
                         │                         │
                         ▼◄────────────────────────┘
                ╭─────────────────╮
                │       END        │
                ╰─────────────────╯
```

24

# FIG.5

# FIG.6

```
        ┌─────────────────┐
        │      START       │
        └────────┬─────────┘
                 │            S11
        ┌────────▼─────────┐
        │ OBTAIN CONPUTED  │
        │FLUORESCENCE VALUE│
        └────────┬─────────┘
                 │            S12
          ╱──────▼──────╲
         ╱ COMPUTED      ╲    NO
        ╱ FLUORESCENCE    ╲──────────┐
        ╲ VALUE ≦ Th1?   ╱           │
         ╲──────┬──────╱             │
                │ YES                │
                │          S14       │
          ╱─────▼──────╲             │
         ╱ FLUORESCENCE ╲    NO       │
        ╱ IMAGE PIXEL    ╲───────────┤
        ╲ VALUES ≧ Th2? ╱           │
         ╲─────┬──────╱             │
               │ YES                │
               │       S15          │          S13
        ┌──────▼───────┐          ┌──▼──────────┐
        │ EXCEPTIONAL   │          │ NO PROCESS  │
        │DISPLAY PROCESS│          └──────┬──────┘
        └──────┬───────┘                 │
               │◄────────────────────────┘
        ┌──────▼───────┐
        │     END       │
        └──────────────┘
```

# FIG.7

# FIG.8

# FIG.9

START

S21
OBTAIN STANDARD
IMAGE COLOR DATA

S22
OUTSIDE RANGE? — NO

YES

S24
FLUORESCENCE IMAGE
PIXEL VALUES ≧ Th3? — NO

YES

S25
EXCEPTIONAL
DISPLAY PROCESS

S23
NO PROCESS

END

# FIG.10

START

↓ S1

OBTAIN STANDARD
IMAGE COLOR DATA

↓ S2

OUTSIDE RANGE? ——NO——→

↓ YES

S11

OBTAIN CONPUTED
FLUORESCENCE VALUE

↓ S12

COMPUTED FLUORESCENCE
VALUE ≦ Th4? ——NO——→

↓ YES

S4                                          S3

EXCEPTIONAL
DISPLAY PROCESS          NO PROCESS

END

# FIG.11

```
            ┌─────────────┐
            │    START     │
            └─────────────┘
                   │
                   ▼            S41
        ┌──────────────────────┐
        │   OBTAIN STANDARD     │
        │  IMAGE COLOR DATA     │
        └──────────────────────┘
                   │
                   ▼            S42
          ╱────────────────╲          NO
         ╱  OUTSIDE RANGE?   ╲──────────────┐
         ╲                   ╱              │
          ╲────────────────╱               │
                   │ YES                    │
                   ▼            S44         │
          ╱────────────────╲      NO        │
         ╱ FLUORESCENCE IMAGE╲──────────────┤
         ╲ PIXEL VALUE  ≧Th5? ╱             │
          ╲────────────────╱                │
                   │ YES                     │
                   ▼            S45          │
        ┌──────────────────────┐            │
        │   OBTAIN CONPUTED     │            │
        │  FLUORESCENCE VALUE   │            │
        └──────────────────────┘            │
                   │                         │
                   ▼            S46          │
          ╱────────────────╲      NO         │
         ╱COMPUTED FLUORESCENCE╲─────────────┤
         ╲  VALUE ≦ Th6?      ╱              │
          ╲────────────────╱                 │
                   │ YES                      │
                   ▼            S47           ▼         S43
        ┌──────────────────────┐   ┌──────────────────┐
        │    EXCEPTIONAL        │   │   NO PROCESS     │
        │  DISPLAY PROCESS      │   └──────────────────┘
        └──────────────────────┘            │
                   │◄───────────────────────┘
                   ▼
            ┌─────────────┐
            │     END      │
            └─────────────┘
```

# FIG.12

# FIG.13

IR

B

EXCITATION
LIGHT

G

R

214d
214
214c
214b
214e
214a

# FIG.14

227
227a
227b

# FIG.15

NORMAL TISSUE

DISEASED TISSUE

INTENSITY

400 450 500 550 600 650 700 750

WAVELENGTH(nm)

# FIG.16

# FIG.17

# FIG.18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5590660 A
- US 5647368 A
- JP 9308604 A
- JP 10225436 A

- JP 2001157658 A
- US 4773097 A
- US 5769729 A
- JP 7176721 A